(19) Europäisches Patentamt
European Patent Office
Office européen des brevets

(11) **EP 4 435 037 A1**

(12) **EUROPEAN PATENT APPLICATION**
published in accordance with Art. 153(4) EPC

(43) Date of publication:
**25.09.2024 Bulletin 2024/39**

(21) Application number: **23823648.3**

(22) Date of filing: **25.05.2023**

(51) International Patent Classification (IPC):
*C08J 3/12* (2006.01)   *A61K 8/02* (2006.01)
*A61K 8/73* (2006.01)   *A61K 8/85* (2006.01)
*A61Q 1/00* (2006.01)   *A61Q 1/06* (2006.01)
*A61Q 1/10* (2006.01)   *A61Q 17/04* (2006.01)

(52) Cooperative Patent Classification (CPC):
**A61K 8/02; A61K 8/73; A61K 8/85; A61Q 1/00;
A61Q 1/06; A61Q 1/10; A61Q 17/04; C08J 3/12**

(86) International application number:
**PCT/JP2023/019437**

(87) International publication number:
**WO 2023/243349 (21.12.2023 Gazette 2023/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.06.2022   JP 2022097945**

(71) Applicant: **Daicel Corporation
Tokyo 108-8230 (JP)**

(72) Inventors:
• **OMURA, Masaya
Tokyo 108-8230 (JP)**
• **UENO, Mizuki
Tokyo 108-8230 (JP)**

(74) Representative: **Grünecker Patent- und
Rechtsanwälte
PartG mbB
Leopoldstraße 4
80802 München (DE)**

(54) **BIODEGRADABLE SPHERICAL PARTICLES AND PRODUCTION METHDO THEREFOR**

(57)   Biodegradable spherical particles contain a biodegradable polymer as a main component. The biodegradable spherical particles have a particle size variation coefficient CV of 40% or less, and a biodegradation degree on the fifth day in a biodegradability test in accordance with OECD TG301F of 40% or less. A cosmetic composition contains the biodegradable spherical particles. A method for producing the biodegradable spherical particles includes preparing a mixture by mixing a biodegradable polymer, a plasticizer, and a water-soluble polymer; melt-kneading the mixture at 200°C or higher and 280°C or lower to yield a kneaded product; and removing the water-soluble polymer from the kneaded product.

FIG. 1

EP 4 435 037 A1

**Description**

Technical Field

The present disclosure relates to biodegradable spherical particles and a method for producing the biodegradable spherical particles. Specifically, the present disclosure relates to biodegradable spherical particles for use in a cosmetic composition, and to a method for producing the biodegradable spherical particles.

Background Art

Typically, various fine polymer particles are blended in cosmetics for purposes such as improving the spread of the cosmetic, changing the texture, imparting a wrinkle blurring effect, and improving the lubricity of a foundation or the like. In particular, fine particles having high sphericity are excellent in imparting good texture and, depending on the physical properties and shape of the fine particles, can provide a light-scattering (soft-focus) effect. When used in a foundation or the like, such fine particles fill and smooth out the roughness of the skin, and scatter light in various directions, and thus can be expected to have an effect of making wrinkles less noticeable (soft-focus).

Examples of such fine particles that are blended into cosmetic products include fine particles formed of a synthetic polymer, such as polyamide, polymethyl methacrylate (PMMA), polystyrene, polypropylene, or polyethylene. However, in recent years, in consideration of the environment, there has been a demand for fine particles formed from a biodegradable material having necessary characteristics and less environmental load in place of these synthetic polymers.

Patent Document 1 (Japanese Patent No. 6872068 B) discloses resin beads containing cellulose as a main component, wherein the resin beads have a cumulative 50% particle size on a volume basis of 50 $\mu$m or less, a sphericity of 0.7 to 1.0, a degree of surface smoothness of 70 to 100%, a degree of solidity of 50 to 100%, and a 5-day biodegradation rate as measured in accordance with JIS K6950 of 20% or more, and the content of the cellulose in the resin is 90 to 100 mass%.

Patent Document 2 discloses a spherical cellulose powder having an average primary particle size of 5 $\mu$m or less, in which 90 vol% or more of all particles have a primary particle size in a range of 2 to 7 $\mu$m, the ratio (longest diameter/shortest diameter) of a longest diameter to a shortest diameter of the spherical shape is in a range of 1.0 to 2.5, and 20 mass% of a silicon oil paste has a haze value of 70% or more and a total transmittance of 95% or more.

Patent Document 3 describes that a resin component (A) such as a thermoplastic resin is kneaded with a water-soluble auxiliary component (B) to prepare a dispersion, and the auxiliary component (B) is eluted from the dispersion to produce a molded article (e.g., a porous article or spherical particles) containing the resin component (A); and also describes that the resin component (A) is a cellulose derivative, polylactic acid, or the like.

Citation List

Patent Document

> Patent Document 1: JP 6872068 B
> Patent Document 2: JP 2013-221000 A
> Patent Document 3: JP 2004-051942 A

Summary of Invention

Technical Problem

The particulate molded article obtained by the production method described in Patent Document 3 has a low sphericity and is in the form of approximately spherical particles, and its biodegradability is not sufficient.

The fine particles containing cellulose as a main component disclosed in Patent Documents 1 and 2 are excellent in biodegradability. However, since the spherical particles described in Patent Document 2 have low surface smoothness, a good texture cannot be achieved. A cosmetic product containing fine particles having excellent biodegradability may provide a change in feeling of use due to degradation of the fine particles after use. In particular, a user is sensitive to a change in texture. There is a demand for biodegradable fine particles that provide little change in feeling of use when blended in a cosmetic composition. The particles disclosed in Patent Document 1 cannot sufficiently suppress a change in feeling of use.

An object of the present disclosure is to provide biodegradable spherical particles capable of achieving a cosmetic composition that has a good texture and causes no change in feeling of use within a short term, and a method for producing the biodegradable spherical particles.

Solution to Problem

The biodegradable spherical particles according to an embodiment of the present disclosure contain a biodegradable polymer as a main component. The biodegradable spherical particles have a particle size variation coefficient CV of 40% or less, and a biodegradation degree on the fifth day in a biodegradability test in accordance with OECD TG301F of 40% or less as calculated by the following formula:

$$\text{biodegradation degree } (\%) = (\text{BOD} - \text{B})/\text{TOD} \times 100$$

(wherein BOD is a biochemical oxygen demand (mg) by a test substance, B is a biochemical oxygen demand (mg) of a blank, and TOD is a theoretical oxygen demand (mg) by the test substance).

Advantageous Effects of Invention

According to the present disclosure, since the biodegradable spherical particles have a narrow particle size distribution and a substantially uniform particle size, a favorable texture is exhibited. The spherical particles have a low initial degradation rate in a predetermined biodegradability test. In other words, the spherical particles degrade slowly and gently. A cosmetic composition containing the spherical particles avoids deterioration of feeling of use due to rapid degradation of the spherical particles.

Brief Description of Drawings

FIG. 1 is a scanning electron microscope (SEM) image (magnification: 800 times) of particles of Example A-1.
FIG. 2 is a scanning electron microscope (SEM) image (magnification: 5000 times or more) of the particles of Example A-1.
FIG. 3 is a scanning electron microscope (SEM) image (magnification: 800 times) of particles of Comparative Example A-2.
FIG. 4 is a scanning electron microscope (SEM) image (magnification: 5000 times or more) of the particles of Comparative Example A-2.
FIG. 5 is a scanning electron microscope (SEM) image (magnification: 800 times) of the particles of Example A-1 after degradation treatment.
FIG. 6 is a scanning electron microscope (SEM) image (magnification: 15000 times) of the particles of Example A-1 after degradation treatment.
FIG. 7 is a scanning electron microscope (SEM) image (magnification: 800 times) of the particles of Comparative Example A-2 after degradation treatment.
FIG. 8 is a scanning electron microscope (SEM) image (magnification: 5000 times) of the particles of Comparative Example A-2 after degradation treatment.

Description of Embodiments

The present disclosure will be described in detail below with reference to preferred embodiments. The configurations, combinations thereof, and the like in each of the embodiments are an example, and various additions, omissions, substitutions, and other changes may be made as appropriate without departing from the spirit of the present disclosure. The present disclosure is not limited by the embodiments and is limited only by the claims. Each aspect disclosed in the present specification can be combined with any other feature disclosed herein.
In the present specification, "X to Y" indicating a range means "X or more and Y or less", "ppm" means "ppm by weight", and unless otherwise noted, all test temperatures are room temperature (20°C $\pm$ 5°C).

Biodegradable spherical particles

The biodegradable spherical particles (hereinafter may be referred to as "spherical particles") according to an embodiment of the present disclosure are particles containing a biodegradable polymer as a main component. Here, the term "main component" means that a component contained in the largest amount among constituent components of the particle is a biodegradable polymer, and the content thereof is at least 50 wt.%. The term "biodegradable polymer" refers to a polymer that degrades in soil or seawater or in a living body. In the present disclosure, the "polymer" is defined as a compound formed by repetitive bonding of one type of constituent unit or two or more types of constituent units. The polymer may be a synthetic polymer or a naturally occurring polymer as long as the polymer exhibits predetermined biodegradability.

The spherical particles have a particle size variation coefficient CV of 40% or less, and a biodegradation degree on the fifth day in a biodegradability test in accordance with OECD TG301F of 40% or less as calculated by the following formula:

$$\text{biodegradation degree (\%)} = (BOD - B)/TOD \times 100$$

(wherein BOD is a biochemical oxygen demand (mg) by a test substance, B is a biochemical oxygen demand (mg) of a blank, and TOD is a theoretical oxygen demand (mg) by the test substance).

The spherical particles according to an embodiment of the present disclosure are formed from a biodegradable material, and have a very good texture due to the shape and narrow particle size distribution thereof. By blending the spherical particles, it is possible to obtain a high-quality cosmetic composition with less environmental load. Moreover, the spherical particles according to an embodiment of the present disclosure exhibit moderate degradability as compared with particles formed from a known biodegradable material. A cosmetic composition containing the spherical particles causes no change in feeling of use, particularly texture, within a short term. The "texture" in the present specification is a concept including skin feeling and tactile sensation in the case of directly touching the spherical particles, and, for example, in the case of touching a cosmetic composition containing the spherical particles.

The spherical particles according to an embodiment of the present disclosure are biodegradable, and the degradation rate is controlled. The biodegradability of the spherical particles can be evaluated by a biodegradability test in accordance with OECD TG301F. Specifically, a test substance (spherical particles) is dispersed in a culture medium (water), inoculated with a plant species source (activated sludge in a sewage treatment plant), and cultured at 22°C $\pm$ 2°C for 28 days, the amount of oxygen consumed by microorganisms for degradation of the test substance (biochemical oxygen demand) is measured over time, and the ratio of the biochemical oxygen demand to the theoretical oxygen demand by the test substance is determined as a biodegradation degree (%). The biodegradation degree in the present specification is determined under the following conditions: test substance concentration: 100 mg/L, plant species source concentration: 30 mg/L, and a test solution amount: 100 mL. The biodegradation degree (%) is calculated by the following formula.

$$\text{Biodegradation degree (\%)} = (BOD - B)/TOD \times 100$$

(wherein BOD is a biochemical oxygen demand (mg) by a test substance, B is a biochemical oxygen demand (mg) of a blank, and TOD is a theoretical oxygen demand (mg) by the test substance.

According to OECD TG301F, a test substance having a biodegradation degree after 28 days of greater than 60% is usually evaluated as "readily degradable". For example, microcrystalline cellulose exhibits a so-called sigmoid degradation behavior in which a degradation reaction rapidly proceeds for 5 days after the start of the test, and then the degradation rate decreases to reach equilibrium. The inventors of the present disclosure considered that the rapid degradation for 5 days after the start of the test is a cause of a short-term change in the texture of the cosmetic composition containing the spherical particles. Then, it has been found that a short-term change in feeling of use is suppressed by reducing the initial degradation rate of the biodegradable spherical particles and setting the biodegradation degree on the fifth day to 40% or less in the biodegradability test in accordance with OECD TG301F described above. That is, the spherical particles according to an embodiment of the present disclosure are different from known biodegradable resin particles in that the spherical particles have a low initial degradation rate and exhibit mild degradation behavior. From the viewpoint of improving the stability of feeling of use, the biodegradation degree of the spherical particles on the fifth day is preferably 35% or less, and more preferably 30% or less. From the viewpoint of low environmental load, the biodegradation degree on the fifth day is preferably 10% or more.

When the biodegradation degree on the fifth day as measured by the biodegradability test in accordance with OECD TG301F is denoted by BD5 (%), and the biodegradation degree on the twenty-eighth day is denoted by BD28 (%), a ratio BD5/BD28 in the spherical particles may be 0.60 or less, 0.55 or less, 0.50 or less, or 0.45 or less, from the viewpoint of having high biodegradability while suppressing a change in feeling of use. From the viewpoint of improving the biodegradability, the ratio BD5/BD28 is preferably 0.10 or more.

It is preferable that the biodegradation degree BD5 on the fifth day and the biodegradation degree BD28 on the twenty-eighth day as measured by the biodegradability test in accordance with OECD TG301F satisfy the following formula from the viewpoint of having high biodegradability while suppressing a change in feeling of use.

$$(BD28 - BD5)/BD5 \geq 0.50$$

In the spherical particles in which the ratio of the difference (BD28 - BD5) between BD28 and BD5 to BD5; i.e., (BD28 - BD5)/BD5 is 0.50 or more, the degradation reaction for 5 days in the biodegradability test is suppressed, and the degradation reaction proceeds for 5 to 28 days. From the viewpoint of improving the biodegradability, the ratio (BD28 - BD5)/BD5 may be 0.60 or more, 0.70 or more, or 0.80 or more. From the viewpoint of a small influence on the texture at an initial use, the ratio (BD28 - BD5)/BD5 is preferably 2.0 or less.

In the spherical particles, the biodegradation degree BD5 on the fifth day and the biodegradation degree BD28 on the twenty-eighth day as measured by the biodegradability test in accordance with OECD TG301F may satisfy the following formula.

$$(BD28 - BD5)/BD28 \geq 0.30$$

In the spherical particles in which the ratio of the difference (BD28 - BD5) between BD28 and BD5 to BD28; i.e., (BD28 - BDS)BD28 is 0.30 or more, high biodegradability is achieved while a change in texture at the initial use is suppressed. From this viewpoint, the ratio (BD28 - BD5)/BD28 may be 0.35 or more, 0.40 or more, or 0.45 or more, and 1.0 or less, or 0.90 or less.

The particle size variation coefficient CV of the spherical particles is 40% or less. In the spherical particles having a particle size variation coefficient CV of 40% or less, a variation in particle size is small. The spherical particles have good texture. In addition, since the spherical particles have a small variation in particle size, the degradation reaction proceeds substantially uniformly in the particles. Thus, even when the degradation reaction proceeds to reduce the particle size, a narrow particle size distribution is maintained. The texture of the spherical particles according to an embodiment of the present disclosure is not deteriorated within a short term. From the viewpoint of physical property stabilization, the particle size variation coefficient CV of the spherical particles is preferably 38% or less, and preferably 35% or less. From the viewpoint of easy production, the particle size variation coefficient CV of the spherical particles may be 0% or more, or 2% or more. The particle size variation coefficient CV is calculated by the following formula using the average particle size of the spherical particles described below and the standard deviation of the particle size.

Particle size variation coefficient CV (%) = standard deviation of particle size/average particle size $\times$ 100

The average particle size of the spherical particles may be 0.08 $\mu$m or more, 0.1 $\mu$m or more, 1.0 $\mu$m or more, 2.0 $\mu$m or more, or 4.0 $\mu$m or more. The average particle size of the spherical particles may be 100 $\mu$m or less, 80 $\mu$m or less, 40 $\mu$m or less, 20 $\mu$m or less, or 10 $\mu$m or less. The particles having an excessively large average particle size may have poor texture and a reduced light-scattering (soft-focus) effect. Alternatively, the particles having an excessively small average particle size may be difficult to produce.

The average particle size and the particle size variation coefficient can be measured using dynamic light scattering. The average particle size is measured specifically as follows. Firstly, a sample is prepared by adding the spherical particles to pure water to make a concentration of 100 ppm, and forming a pure water suspension using an ultrasonic vibrating device. Subsequently, the volume-frequency particle size distribution is measured by laser diffractometry (using the "Laser Diffraction/Scattering Particle Size Distribution Measuring Device LA-960" available from Horiba Ltd., ultrasonic treatment for 15 minutes, refractive index (1.500, medium (water; 1.333))). The particle size corresponding to 50% of the integrated scattering intensity in the volume-frequency particle size distribution is determined as the average particle size. That is, the average particle size ($\mu$m) in the present specification is a volume-based median size. The particle size variation coefficient is calculated from the median size and the standard deviation of the particle size.

The sphericity of the spherical particles according to an embodiment of the present disclosure is preferably 0.7 or more and 1.0 or less, more preferably 0.8 or more and 1.0 or less, and still more preferably 0.9 or more and 1.0 or less. Particles having a sphericity of less than 0.7 have poor texture, and, for example, a cosmetic composition containing such particles results in a poor skin feeling and a less soft-focus effect.

The sphericity can be measured by the following method. From an image of particles observed with a scanning electron microscope (SEM), 30 particles are randomly selected, the major axis length and the minor axis length of each of the randomly selected particles are measured, the (minor axis length)/(major axis length) ratio of each particle is determined, and then the average value of the (minor axis length)/(major axis length) ratios of the randomly selected particles is defined as the sphericity. The particles can be determined to be closer to a true sphere as the sphericity approaches 1. Details of the measurement method will be described in the Examples below.

While the degree of surface smoothness of the spherical particles according to an embodiment of the present disclosure is 80% or more, the degree of surface smoothness is preferably 85% or more, more preferably 90% or more, still more preferably 95% or more, and particularly preferably 98% or more, and the upper limit is 100%. When the degree of surface smoothness is less than 80%, desired texture cannot be achieved, and in addition, the feeling of use may change within a short term. From the viewpoint of improving the texture and stabilizing the physical properties, the degree of surface smoothness of the spherical particles may be 80 to 100%, 85 to 100%, 90 to 100%, 95 to 100%, or 98% to 100%.

The degree of surface smoothness of the spherical particles can be determined as follows: a scanning electron micrograph of the particles is obtained, recesses and protrusions of the particle surfaces are observed, and the degree of surface smoothness is determined based on the area of recessed portions on the surfaces. Details of the method of measuring the degree of surface smoothness will be described in the Examples below.

The shape of the spherical particles according to an embodiment of the present disclosure is not particularly limited, and it is preferable that the spherical particles have the sphericity and the degree of surface smoothness described above; however, in particular, from the viewpoint of suppressing the initial degradation rate and imparting a mild degradation behavior that does not affect the texture, it is more preferable that the spherical particles have a surface shape with less fine recesses and protrusions. The inventors of the present disclosure have focused on the fact that even particles having the same degree of surface smoothness exhibit different degradation behaviors depending on a difference in surface shape, and have found that fine recesses and protrusions on the particle surface, which cannot be evaluated form the degree of surface smoothness described above, affect the degradability of the particles, particularly the initial degradability. For example, when a field of view of 0.5 mm $\times$ 0.5 mm is observed with a scanning electron microscope at a magnification of 5000 times and there is substantially no particle having a micron-sized recess on the surface thereof, or when a field of view of 0.5 mm $\times$ 0.5 mm is observed at a magnification of 5000 times and there is substantially no spherical particle having a micron-sized protrusion protruding from a virtual circle drawn along an arc of the spherical particle, the initial degradation rate is suppressed and a mild degradation behavior is achieved. Here, the "micron-sized" indicates a range of 0.5 $\mu$m or more and less than 10 $\mu$m, and means that the diameter is about 0.5 to 10 $\mu$m in the case of a substantially circular recess.

Here, the term "substantially no" is specifically defined as a case where when 30 randomly sampled particles are observed in a scanning electron microscope image at a magnification of 5000 times or more, an average of the number of micron-sized recesses observed on the surfaces of spherical particles of 3 or less, and preferably 1 or less. The term "substantially no" is also defined as a case where when 30 randomly sampled particles are observed in a scanning electron microscope image at a magnification of 5000 times or more, an average of the number of micron-sized protrusions protruding from a virtual circle drawn along an arc of a spherical particle of 3 or less, and preferably 1 or less.

The main component of the spherical particles according to an embodiment of the present disclosure may be a biodegradable polymer selected from the group consisting of a polysaccharide, a polysaccharide ester, and an aliphatic polyester. The spherical particles may further contain a biodegradable polymer such as an aliphatic polyol, an aliphatic polycarbonate, or a polyacid anhydride as long as the effects of the present disclosure can be obtained.

The polysaccharide means a polymer compound in which monosaccharides are bonded by glycosidic bonds. As long as the effects of the present disclosure are obtained, the polysaccharide may be a polymer of $\alpha$-glucose or a polymer of $\beta$-glucose. Examples thereof include cellulose, hemicellulose, pullulan, amylose, agarose, chitin, chitosan, carrageenan, pectin, dextrin, starch, collagen, mannan, arabinogalactan, glycogen, inulin, hyaluronic acid, and modified products thereof. Two or more types of polysaccharides may be used in combination. One or two types of polysaccharides selected from cellulose and starch are preferred, and cellulose is more preferred. As long as the effects of the present disclosure are obtained, a commercially available polysaccharide may be used, and a polysaccharide obtained by hydrolyzing a polysaccharide ester described below may be used. For example, cellulose may be a fully saponified product of known cellulose diacetate.

The weight average molecular weight of cellulose is not particularly limited as long as the effects of the present disclosure are obtained. The weight average molecular weight of cellulose may be 10000 or more, 20000 or more, or 30000 or more, and 500000 or less, 400000 or less, or 300000 or less. The weight average molecular weight of cellulose can be measured by size exclusion chromatography (GPC) measurement (GPC-light scattering method) in the same manner as the aliphatic polyester described below.

The polysaccharide ester is a carboxylic acid ester of the polysaccharide described above, and is defined as a compound in which some of hydroxyl groups in the molecular chain are substituted with acyl groups. Esters of one or two types of polysaccharides selected from cellulose and starch are preferred, and cellulose esters are more preferred. Two or more types of polysaccharide esters may be used in combination. Carboxylic acid esters of other polysaccharides not specified herein may be used as long as the effects of the present disclosure are obtained.

The total degree of substitution of the polysaccharide ester used in the spherical particles according to an embodiment of the present disclosure is appropriately selected within a range of more than 0 and 3.0 or less depending on the type of the polysaccharide and the type of the substituent. The total degree of substitution of the polysaccharide ester may be 0.05 or more, 0.1 or more, 0.2 or more, 0.3 or more, or 0.5 or more, and 2.95 or less, 2.80 or less, 2.65 or less, 2.00 or less, 1.50 or less, 1.0 or less, or less than 0.7. The total degree of substitution of the polysaccharide ester can be measured by a known method using $^{13}$C-NMR or $^{1}$H-NMR.

From the viewpoint of easy availability and excellent biodegradability, a preferred polysaccharide ester is a cellulose ester, and a cellulose acylate having an acyl group having 2 or more carbons is more preferred. The number of carbons of the acyl group of the cellulose acylate may be 3 or more, or 4 or more, and 10 or less, or 8 or less. The cellulose acylate may have two or more types of acyl groups as substituents. In the present disclosure, two or more types of cellulose acylates having acyl groups with different numbers of carbons may be used in combination as the biodegradable polymer.

Specific examples of the cellulose acylate according to the present disclosure include cellulose acetate, cellulose propionate, cellulose butyrate, cellulose acetate propionate, and cellulose acetate butyrate. From the viewpoint of the biodegradability and easy availability, cellulose acetate, cellulose acetate propionate, and cellulose acetate butyrate are preferred, and cellulose acetate is more preferred.

Similarly to the polysaccharide ester described above, the total degree of substitution of cellulose acylate is appropriately selected within a range of more than 0 and 3.0 or less; however, from the viewpoint of achieving good biodegradability, for example, the total degree of substitution of cellulose acylate having an acyl group having 2 or more and 10 or less carbons is preferably more than 0 and 1.0 or less. The total degree of substitution of cellulose acylate may be 0.05 or more, 0.1 or more, 0.2 or more, 0.3 or more, or 0.4 or more, and 0.9 or less, 0.8 or less, or less than 0.7. Cellulose acylates having different total degrees of substitution may be used in combination, or a cellulose acylate having a total degree of substitution of more than 1.0 and 3.0 or less may be mixed and used, as long as the effects of the present disclosure can be obtained.

The degree of substitution of the cellulose acylate can be measured by the following method. For example, the measurement can be performed according to the method described in Tezuka (Tezuka, Carbonydr. Res. 273, 83 (1995)) using NMR. That is, a free hydroxyl group of the cellulose acylate is acylated with a carboxylic anhydride in pyridine. The type of the carboxylic anhydride used here should be selected depending on the purpose of the analysis; for example, when a degree of propionyl substitution of cellulose propionate is analyzed, acetic anhydride is suitably used. The resulting sample is dissolved in deuterated chloroform, and the $^{13}$C-NMR spectrum is measured. When a cellulose acylate having a propionyl group or a cellulose acylate having no propionyl group is treated with propionic anhydride and then the degree of propionyl substitution is analyzed, carbonyl carbon signals of the propionyl group appear in the same order of the 2-, 3-, and 6-positions from a high magnetic field in the region from 172 ppm to 174 ppm. The total degree of substitution of the cellulose acylate treated with the carboxylic anhydride by the method of Tezuka or a similar method is 3.0, and thus, if the sum of areas of the carbonyl carbon signals of the acyl group originally included in the cellulose acylate and the carbonyl signals of the acyl group introduced by the treatment with carboxylic anhydride is normalized to 3.0, and the presence ratio of each acyl group at each corresponding position (in other words, area ratio of each signal) is determined, each of the degrees of acyl substitution at the 2-, 3-, and 6-positions of a glucose ring in the cellulose ester can be obtained. It goes without saying, a substituent containing an acyl group that can be analyzed by this method is only a substituent group that does not correspond to the carboxylic anhydride used in the treatment for an analytical purpose. The degree of substitution can be analyzed by $^{1}$H-NMR in addition to $^{13}$C-NMR.

The weight average molecular weight of the cellulose acylate is not particularly limited as long as the effects of the present disclosure are obtained. From the viewpoint of easily achieving a desired shape, the weight average molecular weight of the cellulose acylate is preferably 10000 or more, more preferably 20000 or more, still more preferably 30000 or more. From the viewpoint of high biodegradability and easily achieving a desired spherical shape, the weight average molecular weight of the cellulose acylate is preferably 500000 or less, more preferably 400000 or less, still more preferably 300000 or less. The weight average molecular weight of the cellulose acylate can be measured by size exclusion chromatography (GPC) measurement (GPC-light scattering method) similar to the aliphatic polyester described below.

The type of the aliphatic polyester is not particularly limited, and examples thereof include a polyhydroxyalkanoic acid having, as a repeating unit, a constituent unit obtained by polycondensation of a hydroxyalkanoic acid, and a polymer having, as a repeating unit, a constituent unit obtained by dehydration condensation of an aliphatic dicarboxylic acid and an aliphatic diol, from the viewpoint of a polymer structure.

Examples of the polyhydroxyalkanoic acid include polyglycolic acid, polylactic acid, poly(β-hydroxybutyric acid), poly(β-hydroxyvaleric acid), poly(lactic acid-co-glycolic acid), poly(β-hydroxybutyric acid-co-β-hydroxyvaleric acid), poly(β-propiolactone), and poly(ε-caprolactone). Examples of the polymer of an aliphatic dicarboxylic acid and an aliphatic diol include polyethylene succinate, polybutylene succinate, and poly(butylene succinate-co-butylene adipate). Two or more types of them may be used in combination.

From the viewpoint of easy availability and excellent biodegradability, a preferred aliphatic polyester is one type or two or more types selected from the group consisting of polycaprolactone, polyhydroxybutyric acid, and polylactic acid. Other aliphatic polyesters not specified herein may be used as long as the effects of the present disclosure are obtained.

From the viewpoint of easily achieving a desired particle shape, the weight average molecular weight of the aliphatic polyester is preferably 10000 or more, more preferably 20000 or more, still more preferably 50000 or more. From the viewpoint of excellent biodegradability, the weight average molecular weight of the aliphatic polyester is preferably 5000000 or less, more preferably 1000000 or less, still more preferably 500000 or less, and particularly preferably 250000 or less.

The weight average molecular weight of the aliphatic polyester is determined by performing size exclusion chromatography (GPC) measurement using the following apparatus under the following conditions (GPC-light scattering method).

Apparatus: GPC "SYSTEM-21H", available from Shodex
Solvent: acetone
Column: two columns of GMHxl (Tosoh Corporation), guard column (TSKgel guard column HXL-H available from Tosoh Corporation)
Flow rate: 0.8 ml/min
Temperature: 29°C
Sample concentration: 0.25% (wt/vol)
Injection volume: 100 μl
Detection: MALLS (multi-angle light scattering detector) ("DAWN-EOS" available from Wyatt Technology Corporation)
Reference material for MALLS calibration: PMMA (molecular weight: 27600)

In another aspect of the present disclosure, the spherical particles may contain a plasticizer. In the present disclosure, the plasticizer refers to a compound capable of increasing the plasticity of the biodegradable polymer described above. The type of the plasticizer is not particularly limited, and examples thereof include polyvalent carboxylic acid esters, for example, adipic acid-based plasticizers, including adipic acid esters such as dimethyl adipate, dibutyl adipate, diisostearyl adipate, diisodecyl adipate, diisononyl adipate, diisobutyl adipate, diisopropyl adipate, diethylhexyl adipate, dioctyl adipate, dioctyldodecyl adipate, dicapril adipate, dihexyldecyl adipate, di(ethylene glycol monoalkyl ether) adipate, di(diethylene glycol monomethyl ether) adipate, di(triethylene glycol monomethyl ether) adipate, di(tetraethylene glycol monomethyl ether) adipate, di(pentaethylene glycol monomethyl ether) adipate, di(hexaethylene glycol monomethyl ether) adipate, di(propylene glycol monoalkyl ether) adipate, di(dipropylene glycol monomethyl ether) adipate, di(tripropylene glycol monomethyl ether) adipate, di(tetrapropylene glycol monomethyl ether) adipate, di(pentapropylene glycol monomethyl ether) adipate, di(hexapropylene glycol monomethyl ether) adipate, di(polyethylene glycol monoalkyl ether) adipate, di(polypropylene glycol monoalkyl ether) adipate, diphenyl adipate, dinaphthyl adipate, and dibenzyl adipate; citric acid-based plasticizers, including citric acid esters such as acetyl triethyl citrate, acetyl tributyl citrate, isodecyl citrate, isopropyl citrate, triethyl citrate, triethylhexyl citrate, and tributyl citrate; glutaric acid-based plasticizers, including glutaric acid esters such as diisobutyl glutarate, dioctyl glutarate, and dimethyl glutarate; succinic acid-based plasticizers, including succinate esters such as diisobutyl succinate, diethyl succinate, diethylhexyl succinate, and dioctyl succinate; sebacic acid-based plasticizers, including sebacic acid esters such as diisoamyl sebacate, diisooctyl sebacate, diisopropyl sebacate, diethyl sebacate, diethylhexyl sebacate, and dioctyl sebacate; and phthalic acid-based plasticizers, including phthalic acid esters such as ethyl phthalate, methyl phthalate, diaryl phthalate, diethyl phthalate, diethylhexyl phthalate, dioctyl phthalate, dibutyl phthalate, and dimethyl phthalate. These polyvalent carboxylic acid esters may be mixed polybasic acid esters.

Examples of the plasticizer contained in the spherical particles according to an embodiment of the present disclosure include glycerin-based plasticizers, including glycerin alkyl esters such as triacetin, diacetin, and monoacetin; neopentyl glycol; and phosphoric acid-based plasticizers, including phosphoric acid esters such as trioleyl phosphate, tristearyl phosphate, and tricetyl phosphate. Other examples include di-2-methoxyethyl phthalate, dibutyl tartrate, O-benzoyl ethyl benzoate, ethyl phthalyl ethyl glycolate (EPEG), methyl phthalyl ethyl glycolate (MPEG), N-ethyltoluenesulfonamide, O-cresyl p-toluenesulfonate, triethyl phosphate (TEP), triphenyl phosphate (TPP), and tripropionin. The spherical particles may contain one type of plasticizer or two or more types of plasticizers.

From the viewpoint of having a high effect of plasticizing the biodegradable polymer, a polyvalent carboxylic acid-based plasticizer or a glycerin-based plasticizer is preferred, and one type or two or more types selected from mixed polybasic acid esters or glycerin alkyl esters are more preferred. Examples of the plasticizer for the biodegradable polymer include trade name "DAIFATTY-10" available from Daihachi Chemical Industry Co., Ltd., trade names "BIOCIZER", "RIKEMAL PL-004" and "Poem G-002" available from Riken Vitamin Co., Ltd., and trade names "POLYSIZER" and "MONOSIZER" available from DIC Corporation.

When the spherical particles according to an embodiment of the present disclosure contain a plasticizer, the content of the plasticizer contained in the spherical particles is not particularly limited. For example, the content of the plasticizer in the spherical particles may be more than 0 parts by weight and 120 parts by weight or less, 2 parts by weight or more and 100 parts by weight or less, 10 parts by weight or more and 80 parts by weight or less, or 15 parts by weight or more and 50 parts by weight or less with respect to 100 parts by weight of the biodegradable polymer. The content of the plasticizer in the spherical particles can be determined by $^1$H-NMR measurement.

In still another aspect of the present disclosure, a portion or the entirety of the surfaces of the spherical particles may be coated with an inorganic powder and/or an organic compound having a long-chain alkyl group. Because of the inorganic powder and/or the organic compound having a long-chain alkyl group present on the particle surface, the spherical particles can achieve surface physical properties suitable for a solvent and a preparation used in a cosmetic composition. According to the spherical particles having the inorganic powder and/or the organic compound having a long-chain alkyl group on the surface thereof, high particle dispersibility is achieved in various solvents and preparations, and the texture of the resulting cosmetic composition is improved. The inorganic powder and/or the organic compound having a long-chain alkyl group and the spherical particles may be physically attached to each other or may be chemically bonded to each other.

The particle shape of the inorganic powder is not particularly limited as long as the effects of the present disclosure are obtained, and the particle shape may be, for example, any of a spherical shape, a plate-like shape, a needle-like shape, a granular shape, and an irregular shape. The average particle size of the inorganic powder is preferably smaller than the average particle size of the spherical particles, and may be, for example, 1/3 or less or 1/10 or less of the average particle size of the spherical particles. Here, the average particle size of the inorganic powder and the spherical particles means a volume-based median size.

The type of the inorganic powder is not particularly limited, and examples thereof include titanium oxide, silicon oxide, aluminum oxide, zinc oxide, zirconium oxide, magnesium oxide, boron nitride, silicon nitride, barium sulfate, calcium sulfate, magnesium sulfate, calcium carbonate, magnesium carbonate, talc, mica, kaolin, sericite, mica, vermiculite, hydirite, bentonite, montmorillonite, hectorite, kaolinite, zeolite, ceramic powder, hydroxyapatite, calcium phosphate, silicic acid, aluminum silicate, magnesium silicate, aluminum magnesium silicate, and calcium silicate. Two or more types may be used in combination. One type or two or more types selected from the group consisting of titanium oxide, silicon oxide, aluminum oxide, zinc oxide, and zirconium oxide are preferred from the viewpoint of good adhesion to the spherical particles and achieving good texture.

The type of the organic compound having a long-chain alkyl group is not particularly limited, and for example, the number of carbons of the long-chain alkyl group may be 5 or more, 7 or more, or 8 or more, and 22 or less. The number of carbons of the long-chain alkyl group may be 5 or more and 22 or less, 7 or more and 22 or less, or 8 or more and 22 or less. Two or more types of organic compounds having different alkyl groups may be used in combination.

In the present disclosure, the organic compound having a long-chain alkyl group may be an amino acid derivative. The amino acid derivative is preferably Nε-lauroyl-L-lysine.

In the present disclosure, the organic compound having a long-chain alkyl group may be a cationic surfactant. Examples of the cationic surfactant include a quaternary ammonium salt and/or an amine salt. From the viewpoint of improving the texture and the dispersibility, a quaternary ammonium salt represented by the following general formula (1) is preferred.

$$R^1R^2R^3R^4N^+X^- \qquad (1)$$

(In the formula, $X^-$ is a halogen ion, $R^1$, $R^2$, $R^3$, and $R^4$ are each independently an alkyl group having 1 to 25 carbons which may have a substituent, and at least one of $R^1$, $R^2$, $R^3$, or $R^4$ is an alkyl group having 12 or more carbons.)

Specific examples of the cationic surfactant represented by the formula (1) include stearyltrimethylammonium chloride, distearyldimethylammonium chloride, behenyltrimethylammonium chloride, distearyldiethylammonium chloride, decyltriethylammonium chloride, decyldimethylhydroxyethylammonium chloride, coconut oil trimethylammonium chloride, coconut oil methyl dihydroxyethylammonium chloride, myristyltrimethylammonium chloride, lauryltrimethylammonium chloride, distearyldimethylammonium bromide, and stearyltrimethylammonium bromide.

The organic compound having a long-chain alkyl group may be a metal soap. The metal soap is defined as a non-alkali metal salt of a higher fatty acid. The higher fatty acid is preferably a fatty acid having 12 to 25 carbons, and may be a saturated fatty acid or an unsaturated fatty acid. Examples of such fatty acid soaps include zinc salts, calcium salts, magnesium salts, or aluminum salts of lauric acid, myristic acid, palmitic acid, stearic acid, behenic acid, oleic acid, linoleic acid, α-linolenic acid, and erucic acid. An alkaline earth metal salt of a fatty acid having 12 to 25 carbons is preferred, a zinc salt is more preferred, and zinc stearate is particularly preferred.

The addition amount of the inorganic powder and/or the organic compound having a long-chain alkyl group is preferably 1.0 wt.% or more, more preferably 3.0 wt.% or more, and particularly preferably 5.0 wt.% or more from the viewpoint of achieving surface properties suitable for blending into a cosmetic composition. From the viewpoint that the physical properties of the spherical particles are not inhibited, the addition amount of the inorganic powder and/or the organic compound having a long-chain alkyl group is preferably 50.0 wt.% or less, more preferably 30.0 mass% or less, and particularly preferably 10.0 wt.% or less. When two or more types of inorganic powders and/or organic compounds having a long-chain alkyl group are used in combination, the total amount thereof preferably satisfies the above-mentioned range.

Cosmetic composition

The biodegradable spherical particles according to an embodiment of the present disclosure can be suitably used for various cosmetic compositions. According to the cosmetic composition containing the spherical particles, the recesses and protrusions of the skin are filled in and smoothed out due to the shape of the spherical particles, and light is scattered in various directions, to thereby achieve the effect of making wrinkles less noticeable (soft-focus). Since the spherical particles according to an embodiment of the present disclosure have a narrow particle size distribution and a substantially uniform particle size, unconventionally good texture is imparted to the cosmetic composition. Furthermore, the spherical particles according to an embodiment of the present disclosure have a slower initial degradation rate as compared with known spherical particles formed of a biodegradable polymer. Thus, a change in feeling of use associated with the degradation of the spherical particles is avoided. Even when the degradation reaction of the spherical particles gradually proceeds during use, the narrow particle size distribution of the spherical particles is maintained, and thus a user is less likely to feel a change in texture. This cosmetic composition stably exhibits good texture without causing a change in feeling of use within a short period of time.

Examples of the cosmetic composition include foundations, such as liquid foundation and powder foundation; concealers; sunscreens; makeup bases; lipsticks and lipstick bases; Oshiroi face powders, such as body powders, solid face powders, and face powders; solid powder eyeshadows; wrinkle masking creams; and skin and hair external preparations mainly for cosmetic purposes, such as skin care lotions. The form of the cosmetic composition is not limited. The cosmetic form may be any of a liquid preparation such as an aqueous solution, an emulsion, or a suspension; a semisolid preparation such as a gel or a cream; a powder solid agent such as a powder or a granule; and an oily solid agent. In addition, the cosmetic form may be, for example, an emulsion preparation, such as a cream or a milky lotion; an oil gel preparation, such as a lipstick; a powder preparation, such as a foundation; or an aerosol preparation, such as a hair styling agent. The cosmetic composition containing the spherical particles according to an embodiment of the present disclosure, particularly the liquid foundation has excellent spreadability to the skin, ability to cover pores, and slipperiness.

Method for producing biodegradable spherical particles

The spherical particles according to an embodiment of the present disclosure can be produced by sequentially performing the following steps:

(1) preparing a mixture by mixing a biodegradable polymer, a plasticizer, and a water-soluble polymer,
(2) melt-kneading the prepared mixture at 200°C or higher and 280°C or lower to yield a kneaded product, and
(3) removing the water-soluble polymer from the obtained kneaded product.

In the related art, an emulsion method has been common for producing spherical particles using a polymer material. For example, in an O/W emulsion method, a polymer material is dissolved in an organic solvent to form an oil phase, and the oil phase is emulsified and dispersed in an aqueous phase, to thereby elute the organic solvent from a droplet-like oil phase and to form polymer particles. According to this O/W emulsion method, recesses and protrusions inevitably caused by an elution route of the organic solvent are formed on the particle surface. In contrast, the spherical particles according to an embodiment of the present disclosure are produced by a melt-kneading method without using an organic solvent. Thus, recesses and protrusions caused by solvent elution are not formed on the particle surface, and the resultant spherical particles have extremely high surface smoothness. Here, the phrase "recesses and protrusions are not formed on the particle surface" means that when a field of view of 0.5 mm × 0.5 mm is observed with a scanning electron microscope at a magnification of 5000 times, there is substantially no spherical particle having a micron-sized recess on the surface thereof, or when a field of view of 0.5 mm × 0.5 mm is observed at a magnification of 5000 times, there is substantially no spherical particle having a micron-sized protrusion protruding from a virtual circle drawn along an arc of the spherical particle. In the present disclosure, the definition of "substantially no" is as described above.

The biodegradable polymer used in the production method according to an embodiment of the present disclosure is one type or two or more types selected from polysaccharides, polysaccharide esters, and aliphatic polyesters. The polysaccharides, polysaccharide esters, and aliphatic polyesters described above for the spherical particles are appropriately selected and used. The polysaccharides, the polysaccharide esters, and the aliphatic polyesters can be produced by known methods. A commercially available biodegradable polymer may be used as long as the effects of the present disclosure are obtained. A polysaccharide may be obtained by hydrolyzing a polysaccharide ester by a known method. For example, when the biodegradable polymer is a polysaccharide ester, a kneaded product is subjected to an alkali treatment in a step of removing a water-soluble polymer from the kneaded product, to completely saponify the polysaccharide ester, whereby spherical particles containing a polysaccharide as a main component can be produced. Details of the alkali treatment will be described below.

When a cellulose acylate having a total degree of substitution of more than 0 and 3.0 or less is used as the polysaccharide ester, the cellulose acylate is prepared through activating a raw material pulp (cellulose); acylating the activated cellulose with an esterifying agent (acylating agent); deactivating the acylating agent after completion of the acylation reaction; and aging (saponifying, hydrolyzing) the produced cellulose acylate to adjust the total degree of substitution to a desired value. The method may include, prior to the activation step, a pretreatment step; i.e., disintegrating and grinding the raw material pulp, and then spraying and mixing acetic acid with the resultant pulp. The method may include a post-treatment step after the aging (saponifying, hydrolyzing) step; i.e., separation by precipitation, purification, stabilization, and drying.

The total degree of substitution of the cellulose acylate can be adjusted by controlling the conditions of aging (conditions such as time and temperature). It is also possible to produce completely saponified cellulose by setting the total degree of substitution to substantially 0. The type of substituent can be determined by selection of the esterifying agent. Examples of the substituent include an acetyl group, a propionyl group, and a butyryl group. Two or more types of substituents may also be introduced at a desired degree of substitution depending on the application.

The type of the plasticizer used in the production method according to an embodiment of the present disclosure is not particularly limited as long as it has a plasticizing effect in the melt extrusion of the biodegradable polymer. The plasticizer can be appropriately selected depending on the type and physical properties of the biodegradable polymer to be used. Specifically, one type of the above-described plasticizers to be contained in the spherical particles may be used alone, or two or more types thereof may be used in combination. From the viewpoint that the effect of plasticizing the biodegradable polymer is high, a polycarboxylic acid-based plasticizer or a glycerin-based plasticizer is preferred, and one type or two or more types selected from mixed polybasic acid esters or glycerin alkyl esters are more preferred.

The blended amount of the plasticizer may be more than 0 parts by weight and 120 parts by weight or less, 2 parts by weight or more and 100 parts by weight or less, 10 parts by weight or more and 80 parts by weight or less, or 15 parts by weight or more and 50 parts by weight or less with respect to 100 parts by weight of the biodegradable polymer. When the blended amount of the plasticizer is excessively small, the sphericity of the resulting spherical particles tends to decrease, whereas when the blended amount is excessively large, the particle shape may fail to be maintained, resulting in failure to produce desired spherical particles.

The type of the water-soluble polymer used in the production method according to an embodiment of the present disclosure is not particularly limited. Here, the "water-soluble" polymer means that an insoluble content is less than 50 wt.% when 1 g of the polymer is dissolved in 100 g of water at 25°C. In the present disclosure, the water-soluble polymer preferably has thermoplasticity. The "thermoplasticity" means a property of softening in a heated state to exhibit fluidity and solidifying by cooling.

Examples of the water-soluble polymer include polyvinyl alcohol, polyethylene glycol, sodium polyacrylate, polyvinylpyrrolidone, polypropylene oxide, polyglycerin, polyethylene oxide, polyvinyl acetate, modified starch, thermoplastic starch, methyl cellulose, ethyl cellulose, hydroxyethyl cellulose, and hydroxypropyl cellulose. Thermoplastic starch can be prepared by a known method. For example, thermoplastic starch can be produced with reference to JP H06-6307 B or WO 92/04408 by mixing tapioca starch with approximately 20% of glycerin as a plasticizer, and then kneading the mixture with a twin-screw extruder.

In the production method according to an embodiment of the present disclosure, the water-soluble polymer is preferably one type or two or more types selected from the group consisting of polyvinyl alcohol, sodium polyacrylate, polyvinylpyrrolidone, and thermoplastic starch, and more preferably one type or two or more types selected from the group consisting of polyvinyl alcohol and thermoplastic starch. The weight average molecular weight of polyvinyl alcohol is preferably 500 or more and 50000 or less.

The blended amount of the water-soluble polymer is preferably 110 parts by weight or more and 15000 parts by weight or less, more preferably 180 parts by weight or more and 1200 parts by weight or less, still more preferably 200 parts by weight or more and 800 parts by weight or less with respect to 100 parts by weight of the biodegradable polymer. When the blended amount of the water-soluble polymer is less than 110 parts by weight, irregular particles having low surface smoothness may be formed. When the blended amount exceeds 15000 parts by weight, the resulting spherical particles may have an excessively small particle size.

As long as the effects of the present disclosure are obtained, the biodegradable polymer, the plasticizer, and the water-soluble polymer may be mixed in one stage or in multiple stages. The biodegradable polymer, the plasticizer, and the water-soluble polymer may be mixed by melt-kneading. For example, the biodegradable polymer may be mixed or melt-kneaded with the plasticizer to prepare a first mixture, and then the first mixture may be blended with the water-soluble polymer, followed by mixing or melt-kneading.

The mixing of the biodegradable polymer and the plasticizer, or the mixing of the biodegradable polymer, the plasticizer, and the water-soluble polymer can be performed in a dry or wet process using a mixer such as a Henschel mixer. In the case of using a mixer such as a Henschel mixer, the temperature in the mixer is preferably such a temperature that the biodegradable polymer does not melt or degrade, for example, a temperature in a range of 20°C or higher and lower than 200°C.

When the mixing of the biodegradable polymer and the plasticizer or the mixing of the biodegradable polymer, the plasticizer, and the water-soluble polymer is performed by melt-kneading, the mixing may be performed at a temperature in a range of 20°C or higher and lower than 200°C using a mixer such as a Henschel mixer, followed by melt-kneading. When the biodegradable polymer and the plasticizer, or the biodegradable polymer, the plasticizer, and the water-soluble polymer are more homogeneously mixed together within a short period of time, the finally prepared spherical particles have high surface smoothness and provide improved texture and touch feeling.

The melt-kneading may be carried out by thermal mixing with an extruder. The kneading temperature (cylinder temperature) of the extruder may be in a range of 200°C to 230°C. The melt-kneading performed at a temperature in this range enables plasticization to provide a uniform kneaded product. When the kneading temperature is excessively low, the resultant particles may have low sphericity and surface smoothness, resulting in poor texture and optical properties. When the kneading temperature is excessively high, the kneaded product may be denatured or colored by heat. A decrease in the viscosity of the melted product due to a high kneading temperature may lead to insufficient kneading of the resin in a twin-screw extruder.

For example, when cellulose acylate is used as the biodegradable polymer, the kneading temperature (cylinder temperature) of the twin-screw extruder may be 200°C. The kneaded product may be extruded into a strand shape and then cut into a pellet shape. In this case, the die temperature may be approximately 220°C.

In the production method according to an embodiment of the present disclosure, a mixture containing the biodegradable polymer, the plasticizer, and the water-soluble polymer is melt-kneaded at 200°C or higher and 280°C or lower to prepare a kneaded product. When the above-described biodegradable polymer, plasticizer, and water-soluble polymer are mixed by melt-kneading at 200°C or higher and 280°C or lower, the kneaded product prepared by the mixing may be directly subjected to the subsequent step.

An extruder such as a twin-screw extruder can be used for melt-kneading the mixture. When an extruder is used, the kneading temperature means the cylinder temperature. The kneaded product containing the biodegradable polymer may be extruded from a die attached to a tip of the extruder into a string shape and then cut into pellets. In this case, the die temperature may be 220°C or higher and 300°C or lower.

The melt-kneading of a mixture containing the biodegradable polymer, the plasticizer, and the water-soluble polymer at 200°C or higher and 280°C or lower produces a dispersion containing the water-soluble polymer as a dispersion medium and a mixture of the biodegradable polymer and the plasticizer as a dispersoid. In other words, the kneaded product according to an embodiment of the present disclosure is a dispersion in which substantially spherical particles containing the biodegradable polymer and the plasticizer are dispersed in a matrix composed of the water-soluble polymer.

The removal of the water-soluble polymer from the kneaded product produces spherical particles containing the biodegradable polymer as a main component and having a particle size variation coefficient CV of 40% or less and a biodegradation degree on the fifth day in a biodegradability test in accordance with OECD TG301F of 40% or less.

Examples of the method of removing the water-soluble polymer include a method of removing the water-soluble polymer by bringing the kneaded product after pressurization into contact with a good solvent of the water-soluble polymer and eluting the water-soluble polymer into the solvent. Examples of the solvent include water; alcohols such as methanol, ethanol, and isopropanol; and mixed solvents thereof. Specifically, the water-soluble polymer can be removed from the kneaded product after pressurization by mixing the kneaded product after pressurization with the solvent, eluting the water-soluble polymer into the solvent, and then removing a filtered product by filtration.

In the process of removing the water-soluble polymer from the kneaded product after pressurization, the plasticizer may be or may not be removed together with the water-soluble polymer. Therefore, the resulting spherical particles may or may not contain the plasticizer.

From the viewpoint of high removal efficiency of the water-soluble polymer, in mixing proportions of the kneaded product and the solvent, the content of the kneaded product is preferably 0.01 wt.% or more and 20 wt.% or less, more preferably 2 wt.% or more and 15 wt.% or less, and still more preferably 4 wt.% or more and 13 wt.% or less with respect to the total weight of the kneaded product and the solvent. When the content of the kneaded product is more than 20 wt.%, the water-soluble polymer may not be sufficiently removed. It may be difficult to separate a solid component containing the spherical particles from a liquid component containing the dissolved water-soluble polymer by an operation such as filtration or centrifugation.

From the viewpoint of high removal efficiency of the water-soluble polymer, the mixing temperature of the kneaded product and the solvent is preferably 0°C or higher and 200°C or lower, more preferably 20°C or higher and 110°C or lower, still more preferably 40°C or higher and 80°C or lower. When the mixing temperature is lower than 0°C, the water-soluble polymer may not be sufficiently dissolved and may be difficult to remove. Meanwhile, when the mixing temperature exceeds 200°C, it may be difficult to achieve a desired particle shape due to deformation or aggregation of particles.

The mixing time of the kneaded product and the solvent is not particularly limited, and may be appropriately adjusted. For example, the mixing time may be 0.5 hours or more, 1 hour or more, 3 hours or more, or 5 hours or more, and 6 hours or less.

A stirring device such as an ultrasonic homogenizer or Three-One Motor can be used for a method of mixing the kneaded product and the solvent to elute the water-soluble polymer. For example, when Three-One Motor is used as the stirring device, the rotation speed during mixing of the kneaded product and the solvent may be 5 rpm or more and 3000 rpm or less. Thus, the water-soluble polymer can be efficiently removed from the kneaded product. The plasticizer can also be efficiently removed from the kneaded product.

When the biodegradable polymer is a polysaccharide ester, as described above, the kneaded product may be subjected to an alkali treatment in the step of removing the water-soluble polymer from the kneaded product. The polysaccharide ester in the kneaded product is saponified by the alkali treatment, to produce a polysaccharide ester having a desired degree of substitution. The alkali treatment under a condition of complete saponification can produce a polysaccharide having a degree of substitution of 0.

The alkali treatment is performed by mixing the kneaded product with a solvent containing one or two or more types of metal compounds selected from an alkali metal compound and an alkaline earth metal compound in the step of removing the water-soluble polymer from the kneaded product. In other words, when the biodegradable polymer is a polysaccharide ester, this production method further includes mixing the kneaded product with a solvent containing one or two or more types of metal compounds selected from an alkali metal compound and an alkaline earth metal compound to hydrolyze the polysaccharide ester. This solvent may be an aqueous solution of one or two or more types of metal compounds selected from an alkali metal compound and an alkaline earth metal compound. The washing of the kneaded product with the solution containing the metal compound can remove the water-soluble polymer and also can provide the polysaccharide ester with a desired degree of substitution.

Examples of the alkali metal compound and the alkaline earth metal compound include compounds of alkali metals such as sodium, lithium, and potassium, and compounds of alkaline earth metals such as calcium, magnesium, and barium. A hydroxide, oxide, or carbonate of an alkali metal or an alkaline earth metal is preferred, and a hydroxide of an alkali metal or an alkaline earth metal is more preferred. Examples of such a metal compound include sodium hydroxide, magnesium hydroxide, and calcium hydroxide.

The addition amount of the metal compound is appropriately selected depending on the type of the polysaccharide ester and the degree of substitution. When the stirring and mixing of the kneaded product and the solvent and the filtration are repeated a plurality of times to remove the water-soluble polymer, the washing with the metal compound-containing solution is preferably implemented at least once, and the washing with the metal compound-containing solution is more preferably implemented at the end of the removal step.

The production method according to another embodiment of the present disclosure may include adding an inorganic powder to and mixing it with spherical particles produced by removing the water-soluble polymer from the kneaded product. This method produces spherical particles in which a portion or the entirety of the surfaces is coated with the inorganic powder. The spherical particles have further improved texture.

The inorganic powder is preferably one type or two or more types selected from the group consisting of titanium oxide, silicon oxide, aluminum oxide, zinc oxide, and zirconium oxide from the viewpoint of achieving good texture. The addition amount of the inorganic powder is preferably 0.01 parts by weight or more and 1.0 part by weight or less with respect to 100 parts by weight of the biodegradable polymer.

The method of adding the inorganic powder to and mixing it with the spherical particles produced by removing the water-soluble polymer is not particularly limited, and a known mixing means is appropriately selected and used. The method may involve dry mixing or wet mixing. For example, the dry mixing may involve the use of a mixing device such as a ball mill, a sand mill, a bead mill, a homogenizer, a planetary mixer, or FII,MIX. The order of mixing the spherical particles and the inorganic powder is not particularly limited. The spherical particles and the inorganic powder may be simultaneously charged into the mixing device, or a predetermined amount of the inorganic powder may be charged into the mixing device and stirred (or pulverized simultaneously with stirring), and then the spherical particles may be charged and mixed.

If necessary, the production method according to an embodiment of the present disclosure may include drying the produced spherical particles after removing the water-soluble polymer and/or adding and mixing the inorganic powder. The drying method is not particularly limited, and a known method such as heat drying, reduced-pressure drying, or vacuum drying can be used. From the viewpoint of high drying efficiency, the drying temperature is preferably room temperature or higher, and may be 50°C or higher or 60°C or higher. From the viewpoint of suppressing thermal degradation, the drying temperature is preferably 120°C or lower.

Examples

The present disclosure will next be specifically described with reference to Examples, but the technical scope of the present disclosure is not limited by these Examples. The configurations and combinations thereof in each of the embodiments are an example, and various additions, omissions, substitutions, and other changes may be made as appropriate without departing from the spirit of the present disclosure.

Example A-1

100 parts by weight of cellulose acetate (available from Daicel Corporation, total degree of substitution = 2.4) as a biodegradable polymer was blended with 25 parts by weight of triacetin (available from Daicel Corporation) as a plasticizer in a dry state, and the blend was dried at 80°C for 12 hours or more. The blend was further stirred and mixed using a Henschel mixer to prepare a mixture of the biodegradable polymer and the plasticizer. The resultant mixture was fed to a twin-screw extruder (PCM30 available from Ikegai Corp., cylinder temperature: 200°C, die temperature: 220°C), melt-kneaded, and extruded to thereby prepare pellets.

30 parts by weight of the pellets were blended with 70 parts by weight of polyvinyl alcohol (available from The Nippon Synthetic Chemical Industry Co., Ltd., a melting point of 190°C, a saponification degree of 99.1%) as a water-soluble polymer in a dry state, and then the blend was fed to a twin-screw extruder (PCM30 available from Ikegai Corp., a cylinder temperature of 220°C, a die temperature of 220°C), melt-kneaded, and extruded to thereby prepare a kneaded product containing the biodegradable polymer, the plasticizer, and the water-soluble polymer.

Separately, sodium hydroxide (available from NACALAI TESQUE, INC.) was dissolved in pure water as a solvent, to prepare an aqueous sodium hydroxide solution having a concentration of 4.7 wt.%.

Next, the resulting kneaded product was mixed with the aqueous sodium hydroxide solution (solvent) described above to achieve a concentration of 5 wt.% (= weight of kneaded product/(weight of kneaded product + weight of solvent) $\times$ 100) or less, and the mixture was stirred using Three-One Motor (BL-3000 available from Shinto Scientific Co., Ltd.) at a rotation speed of 100 rpm and at a temperature of 80°C for 3 hours. The solution after stirring was filtered with filter paper (No. 5A available from ADVANTEC), and the filtered product was removed. The removed filtered product was again mixed with pure water such that a kneaded product concentration was 5 wt.% or less, and the mixture was further stirred at a temperature of 80°C and a rotation speed of 100 rpm for 3 hours. After filtration, the operation of stirring the filtered product in water was repeated three times or more to produce spherical particles of Example A-1.

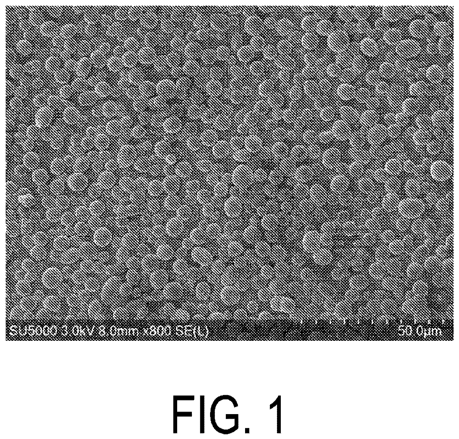

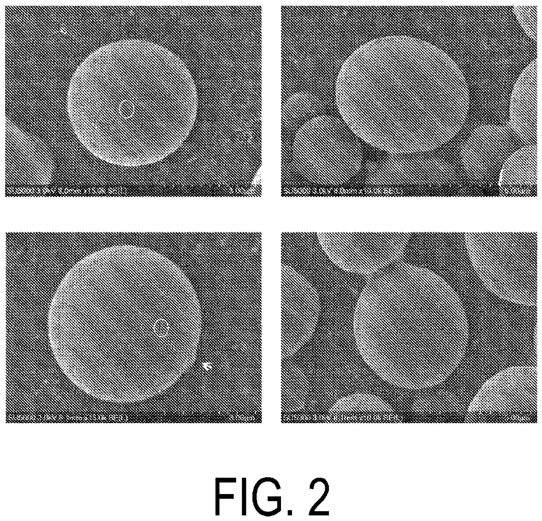

As a result of $^1$H-NMR analysis of the resulting spherical particles, it was confirmed that the degree of acetyl substitution was 0.01, and the main component of the spherical particles of Example A-1 was substantially cellulose. A sample of Example A-1 was observed using a scanning electron microscope (trade name "TM 3000" available from Hitachi High-Technologies Corporation). FIG. 1 is an example of an SEM image of the sample of Example A-1 taken at a magnification of 800 times. The length of a scale bar in FIG. 1 is 50.0 $\mu$m. A field of view of 0.5 mm $\times$ 0.5 mm was observed at a magnification of 5000 times, and it was confirmed that there was substantially no particle having a micron-sized recess observed on the surface thereof and there was substantially no spherical particle having a micron-sized protrusion protruding from a virtual circle drawn along an arc of the spherical particle. The results are shown in Table 1 below as "SEM observation (magnification of 5000 times)". In addition, the number of micron-sized recesses and the number of micron-sized protrusions were measured for 30 randomly sampled particles in an SEM image at a magnification of 5000 times or more, and the numbers were averaged. As a result, the average values of the number of micron-sized recesses and the number of micron-sized protrusions were 0.50 and 0.47, respectively. A portion of an SEM image of the sampled spherical particles is illustrated in FIG. 2. In each image of FIG. 2, a portion surrounded by a circle corresponds to a micron-sized recess, and a portion indicated by an arrow corresponds to a micron-sized protrusion protruding from a virtual circle drawn along an arc of a substantially spherical particle.

The average particle size ($\mu$m), particle size variation coefficient (%), sphericity (%), and degree of surface smoothness (%) of the spherical particles of Example A-1 were measured, and the spherical particles were evaluated for biodegradability and texture. Regarding the texture, texture 1 immediately after production and texture 2 after degradation treatment were evaluated. The evaluation results are shown in Table 1. The measurement or evaluation of the average particle size, the particle size variation coefficient, the sphericity, the degree of surface smoothness, the biodegradability, the texture 1, and the texture 2 were performed by the following methods.

Average particle size and particle size variation coefficient CV

The particles were added to pure water so that the concentration was adjusted to approximately 100 ppm, and a suspension was prepared using an ultrasonic vibrator. Subsequently, the volume-frequency particle size distribution was determined by laser diffractometry ("Laser Diffraction/Scattering Particle Size Distribution Measuring Device LA-960" available from Horiba Ltd., ultrasonic treatment for 15 minutes, refractive index (1.500, medium (water; 1.333))), and the average particle size was measured. The average particle size ($\mu$m) was the value of the particle size corresponding to 50% of the integrated scattering intensity in the volume-frequency particle size distribution (volume-based median size). The particle size variation coefficient CV (%) was calculated by the following formula: standard deviation of particle size/average particle size $\times$ 100. The obtained results are shown in Table 1 below.

Sphericity

Using an image of particles observed with a scanning electron microscope (SEM), the major axis lengths and the minor axis lengths of 30 randomly selected particles were measured to determine the (minor axis length)/(major axis length) ratio of each particle, and the average value of the (minor axis length)/(major axis length) ratios was defined as the sphericity. The obtained results are shown in Table 1 below.

Degree of surface smoothness

An image of the particles observed with a scanning electron microscope (SEM) was binarized using an image processor Winroof (available from Mitani Corporation). A region including the center and/or a vicinity of the center of one particle was randomly selected from the binarized image. The area ratio of a portion (shade portion) corresponding to recesses of unevenness in the region was calculated, and the degree of surface smoothness (%) of the one particle was calculated by the following formula:

Degree of surface smoothness of one particle (%) = (1 - the area ration of recesses) $\times$ 100;

 and

Area ratio of recesses = the area of recesses in the randomly selected region/the area of the randomly selected region.

The average value of the degrees of surface smoothness of ten randomly selected particle samples (n1 to n10) was defined as a degree of surface smoothness (%). The larger the value, the higher the degree of surface smoothness of the particles. The region used for calculating the area ratio may be any region smaller than one particle, including the center and/or a vicinity of the center of the one particle. The size of the region may be 5 $\mu$m square when the particle size is 15 $\mu$m.

Biodegradability

In accordance with OECD TG301F, a biodegradability test of particles was performed under the following conditions.

Inoculum source: return sludge from urban sewage treatment plant
Target substance: microcrystalline cellulose
Test substance concentration: 100 mg/L
Target substance concentration: 100 mg/L
Inoculum source concentration: 30 mg/L
Test solution amount: 100 mL
Test temperature: 22°C $\pm$ 2°C
Culture period: 28 days

Thereafter, the biodegradation degree BD5 (%) after 5 days and the biodegradation degree BD28 (%) after 28 days were calculated by the following formula. The obtained results are shown in Table 1 below.

$$\text{Biodegradation degree (\%)} = (\text{BOD - B})/\text{TOD} \times 100$$

(wherein BOD is the biochemical oxygen demand (mg) by a test substance (particles), B is the biochemical oxygen demand (mg) of a blank, and TOD is a theoretical oxygen demand (mg) by the test substance.)

Texture 1

Sensory evaluation was performed by a panel test of 20 persons for the texture of the particles immediately after production. The persons were instructed to touch the particles to evaluate comprehensively smoothness and moist feeling as the texture, on a scale with a maximum score of 5 points according to the following criteria. The results of calculating average scores of the 20 persons are shown as "Texture 1" in Table 1 below.
Good: 5, Slightly good: 4, Fair: 3, Slightly poor: 2, and Poor: 1.

Texture 2

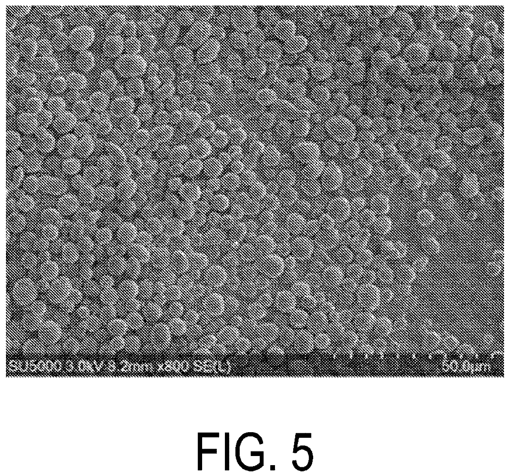

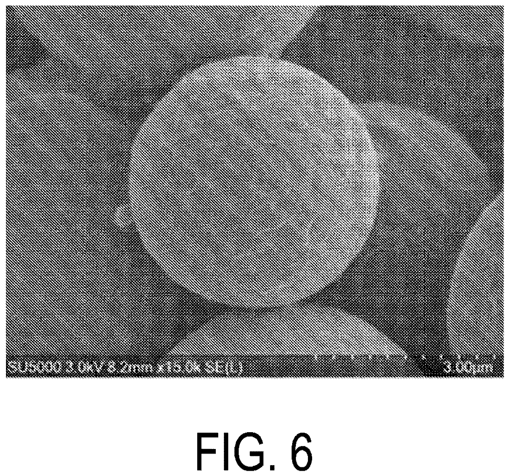

Assuming that bacteria were mixed in a cosmetic composition containing the particles for some reason, cellulase was simulatively mixed to perform a degradation treatment, and a change in texture of the particles before and after degradation was evaluated.
Firstly, 3.0 g of the particles were collected in a screw tube having a volume of 100 mL, and 0.0030 g of cellulase (available from MP Biomedicals) and 30 mL of a buffer solution (pH 5.0) were added. After immersion in a thermostatic chamber set at 40°C for 24 hours, a supernatant was removed, and dried at 80°C using a vacuum dryer to yield particles after the degradation treatment. FIGS. 5 and 6 are examples of SEM images taken at magnifications of 800 times and 15000 times, respectively, of the particles of Example A-1 after the degradation treatment. The length of the scale bar in FIG. 5 is 50.0 μm, and the length of the scale bar in FIG. 6 is 3.00 μm.
The particles before the degradation treatment or the particles after the 24-hour degradation treatment were collected (5.0 g), and the particles were added to 100 mL of water and stirred, to thereby prepare a suspension. The suspension was used, and sensory evaluation before and after the degradation treatment was performed by a panel test of 20 persons. The persons were instructed to use the suspension to evaluate comprehensively both smoothness and moist feeling, on a scale with a maximum score of 5 points according to the following criteria. The results of calculating average scores of the 20 persons are shown as "Texture 2 (before degradation/after degradation)" in Table 1 below.
Good: 5, Slightly good: 4, Fair: 3, Slightly poor: 2, and Poor: 1.

Examples A-2 to A-4

Spherical particles of Examples A-2 to A-4 were produced in the same manner as in Example A-1 except that the concentrations of the aqueous sodium hydroxide solution were changed to 4.1 wt.%, 3.7 wt.%, and 3.2 wt.%, respectively. Table 1 below shows a total degree of substitution DS measured by $^1$H-NMR analysis, the physical properties of the particles of each of the Examples evaluated by the above-described methods, and the results of SEM observation. In Table 1, "Absence" in "SEM observation (5000 times)" means that when a field of view of 0.5 mm × 0.5 mm was observed at a magnification of 5000 times, there was substantially no particle having a micron-sized recess or protrusion on the surface.

Examples A-5 and A-7

Spherical particles of Examples A-5 to A-7 were produced in the same manner as in Example A-1 except that the biodegradable polymer was changed as shown in Table 2 below and distilled water was used instead of the sodium hydroxide solution. Table 2 below shows the average particle size (μm), particle size variation coefficient (%), sphericity (%), degree of surface smoothness (%), biodegradability, and texture 1 of the particles of each of the Examples evaluated by the above-described methods, and the results of SEM observation. In Table 2, "Absence" in "SEM observation (5000 times)" means that when a field of view of 0.5 mm × 0.5 mm was observed at a magnification of 5000 times, there was substantially no particle having a micron-sized recess or protrusion on the surface.

Comparative Example A-1

Commercially available microcrystalline cellulose (trade name "Ceolus-PH-101" available from Asahi Kasei Corporation) was used as particles of Comparative Example A-1. The particles of Comparative Example A-1 were used as a target substance and subjected to the biodegradability test described above. Table 2 below shows the average particle size (μm), the particle size variation coefficient (%), the sphericity (%), the degree of surface smoothness (%), the biodegradability, and the texture 1 evaluated by the above-described methods, and the results of SEM observation. In Table 2, "Unmeasurable" in "SEM observation (5000 times)" means that the surface shape could not be evaluated because of irregular particle shape.

Comparative Example A-2

250 Parts by weight of cellulose acetate (available from Daicel Corporation, total degree of acetyl substitution: 2.4) was dissolved in 2250 parts by weight of acetone (available from NACALAI TESQUE, INC.) to prepare a cellulose acetate solution. In addition, 200 parts by weight of polyvinyl alcohol (available from Kuraray Co., Ltd.) was dissolved in 2300 parts by weight of ion-exchanged water to prepare a medium phase. The entire amount of the cellulose acetate solution was added to the prepared medium phase and mixed, and the mixture was stirred at 3000 rpm for 3 minutes using a homodisperser, and then further stirred at 2000 rpm for 10 minutes to prepare a suspension in which the cellulose acetate solution was uniformly dispersed in droplet form. While the suspension was stirred at 500 rpm using the homodisperser, 112500 parts by weight of ion-exchanged water was injected over 75 minutes to prepare a dispersion of cellulose acetate resin particles. The resin particles were filtered and washed, and then dispersed in 2500 parts by weight of ion-exchanged water. Sodium hydroxide was added to the dispersion to adjust the pH to 13.0 or less, and the dispersion was heated to 60°C to perform a hydrolysis reaction. After completion of the hydrolysis reaction, neutralization was performed with hydrochloric acid, and the product was filtered and washed, and then peptized with ion-exchanged water. In addition, after filtration and washing, drying and disintegration were performed to produce particles of Comparative Example A-2.

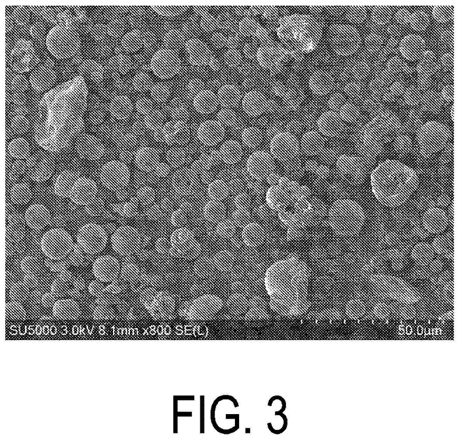

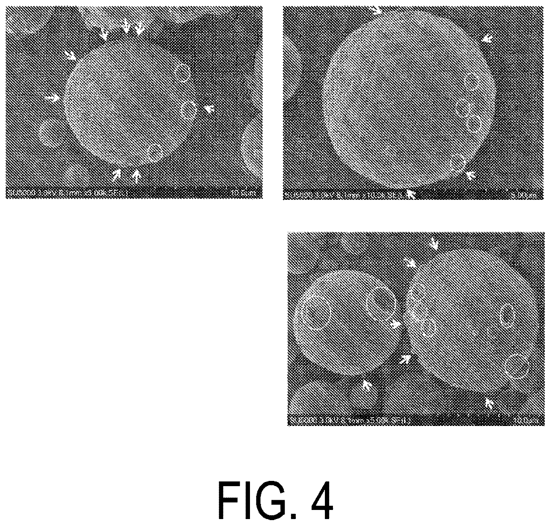

$^1$H-NMR analysis of the produced particles was performed to confirm that the main component of the particles of Comparative Example A-2 was substantially cellulose. Table 2 below shows the average particle size ($\mu$m), the particle size variation coefficient (%), the sphericity (%), the degree of surface smoothness (%), the biodegradability, the texture 1, and the texture 2 evaluated by the above-described methods, and the results of SEM observation. FIG. 3 illustrates an example of an SEM image of the particles Comparative Example A-2 taken at a magnification of 800 times. The length of the scale bar in FIG. 3 is 50.0 $\mu$m. In Table 2, "Presence" in "SEM observation (5000 times)" means that when a field of view of 0.5 mm $\times$ 0.5 mm is observed at a magnification of 5000 times, there are a plurality of particles having a micron-sized recess or protrusion on the surfaces. Specifically, the number of micron-sized recesses and the number of micron-sized protrusions were measured for 30 randomly sampled particles in an SEM image at a magnification of 5000 times or more, and the numbers were averaged. As a result, the average values of the number of micron-sized recesses and the number of micron-sized protrusions were 3.47 and 3.87, respectively. A portion of an SEM image of the sampled spherical particles is illustrated in FIG. 4. In each image of FIG. 4, a portion surrounded by a circle corresponds to a micron-sized recess, and a portion indicated by an arrow corresponds to a micron-sized protrusion protruding from a virtual circle drawn along an arc of a substantially spherical particle.

[Table 1]

|  | Example A-1 | Example A-2 | Example A-3 | Example A-4 |
|---|---|---|---|---|
| Biodegradable polymer | CA0 | CA | CA | CA |
| Total degree of substitution DS | 0 | 0.4 | 0.7 | 1.0 |
| Average particle size ($\mu$m) | 6.2 | 6.7 | 6.2 | 6.4 |
| Particle size variation coefficient CV (%) | 32.7 | 32.7 | 32.3 | 34.4 |
| Sphericity | 1.0 | 1.0 | 1.0 | 1.0 |
| Degree of surface smoothness (%) | 99.0 | 99.0 | 99.0 | 99.0 |
| SEM observation (magnification 5000 times) | Absence | Absence | Absence | Absence |
| Texture 1 | 4.6 | 4.8 | 4.8 | 4.7 |
| Texture 2 (before decomposition/after decomposition) | 4.4/4.1 | - | - | - |
| Biodegradability |  |  |  |  |
| BD5 (%) | 25.8 | 24.2 | 22.4 | 13.9 |
| BD28 (%) | 62.3 | 49.4 | 41.6 | 32.0 |
| BD5/BD28 | 0.41 | 0.49 | 0.54 | 0.43 |
| (BD28 - BD5)/BD5 | 1.41 | 1.04 | 0.86 | 1.30 |

[Table 2]

| | Example A-5 | Example A-6 | Example A-7 | Comparative Example A-1 | Comparative Example A-2 |
|---|---|---|---|---|---|
| Biodegradable polymer | CAP | PHB | PCL | PH101 | CA0 |
| Total degree of substitution DS | 0.4 | - | - | 0 | 0 |
| Average particle size ($\mu$m) | 6.9 | 7.8 | 7.2 | 49.0 | 9.0 |
| Particle size variation coefficient CV (%) | 35.2 | 38.9 | 36.3 | 82.3 | 48.6 |
| Sphericity | 1.0 | 1.0 | 1.0 | 0.5 | 1.0 |
| Degree of surface smoothness (%) | 99.0 | 99.0 | 99.0 | 43.0 | 91.0 |
| SEM observation (magnification 5000 times) | Absence | Absence | Absence | Unmeasurable | Presence |
| Texture 1 | 4.5 | 3.8 | 3.9 | 2.1 | 3.9 |
| Texture 2 (before decomposition/after decomposition) | - | - | - | - | 3.8/3.2 |
| Biodegradability | | | | | |
| BD5 (%) | 21.3 | 11.2 | 12.8 | 32.0 | 49.0 |
| BD28 (%) | 39.6 | 61.3 | 75.1 | 67.0 | 68.0 |
| BD5/BD28 | 0.54 | 0.18 | 0.17 | 0.48 | 0.72 |
| (BD28 - BD5)/BD5 | 0.86 | 4.47 | 4.87 | 1.09 | 0.39 |

The details of the compounds shown in Tables 1 and 2 are as follows.

CA: cellulose acetate available from Daicel Corporation, (total degree of substitution 2.4, weight average molecular weight 47000)

CA0: completely saponified product of cellulose acetate CA (total degree of substitution 2.4, weight average molecular weight 47000) available from Daicel Corporation
CAP: cellulose acetate propionate (available from Eastman Chemical Company: CAP-482-0.5, degree of acetyl substitution = 0.18, degree of propionyl substitution = 2.40)
PCL: polycaprolactone (weight average molecular weight 50000) available from Daicel Corporation
PHB: polyhydroxybutyric acid (weight average molecular weight 550000) available from Goodfellow

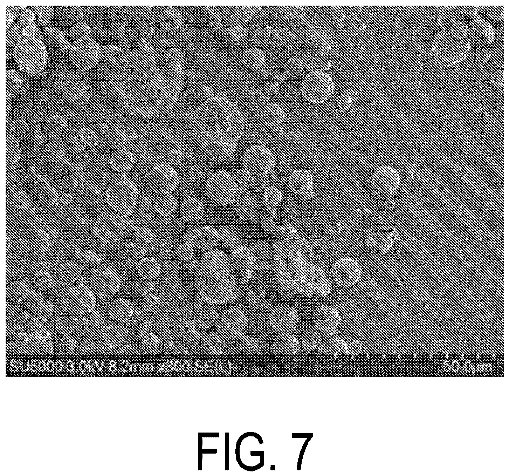

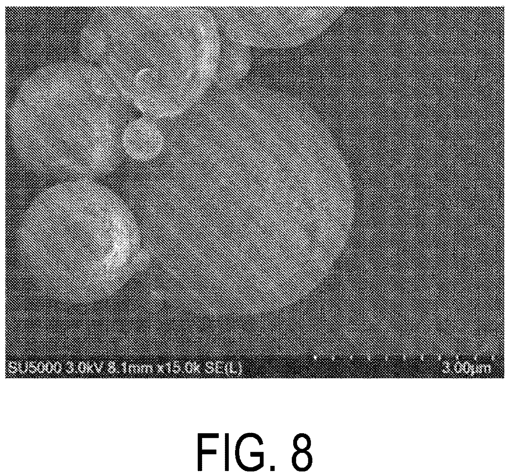

As shown in Tables 1 and 2, it was confirmed that all the spherical particles of the Examples had good texture, and exhibited moderate biodegradability as compared with the particles of the Comparative Examples. As illustrated in FIGS. 1 to 8, in the Examples, the spherical particles were degraded by cellulase from the surfaces, and maintained a substantially spherical shape even after the degradation treatment, and as a result, a change in texture 2 was only a reduction by 0.3 points. In contrast, in the Comparative Examples, the degradation proceeded from a site where recesses and protrusions were remarkable, and non-uniformity of the particle shape increased, and thus the texture 2 was further reduced by 0.6 points.

Example B-1

Preparation of liquid foundation

Components shown in Table 3 were mixed, and then stirred well, and the mixture was charged into a container to prepare a liquid foundation. The texture of the resulting liquid foundation was evaluated by the method described below. The results are shown in Table 11.

[Table 3]

(Table 3) Example B-1

| Component | Product name, etc. | wt.% |
|---|---|---|
| Cyclopentasiloxane | KF-995 (Shin-Etsu Chemical Co., Ltd.) | 15.2 |
| Mineral oil | HICALL K-230 (KANEDA Co., Ltd.) | 5.0 |
| Ethylhexyl methoxycinnamate | Uvinul MC80 (BASF) | 4.0 |
| Isononyl isononanoate | KAK-99 (Kokyu Alcohol Kogyo Co., Ltd.) | 3.0 |
| Disteardimonium hectorite, cyclopentasiloxane, etc. | Bentone Gel VS-5 PC V HV (Elementis) | 3.0 |
| Phytosteryl macadamiate | Plandool-MAS (Nippon Fine Chemical Co., Ltd.) | 0.3 |
| Trimethylsiloxysilicate, polypropylsilsesquioxane | MQ-1640 Flake Resin (Dow Coming Toray Co., Ltd.) | 0.3 |
| PEG-10 dimethicone | KF-6017P (Shin-Etsu Chemical Co., Ltd.) | 1.5 |
| Polyglyceryl oleate-2, polyhydroxystearic acid, polyglyceryl stearate-2 | PolyAquol OS2 (innovacos) | 1.0 |
| Titanium oxide, cyclopentasiloxane, etc. | SDL-Ti70 (Daito Kasei Kogyo Co., Ltd.) | 12.3 |
| Iron oxide, cyclopentasiloxane, etc. | SDL-IOY50 (Daito Kasei Kogyo Co., Ltd.) | 3.0 |
| | SDL-IOR50 (Daito Kasei Kogyo Co., Ltd.) | |
| | SDL-IOB50 (Daito Kasei Kogyo Co., Ltd.) | |
| Example A-1: CA0 spherical particles | | 3.0 |
| BG | 1,3-BG (UK) (Daicel Corporation) | 6.0 |
| Phenoxyethanol | Phenoxyethanol-SP (Yokkaichi Chemical Co., Ltd.) | 0.3 |
| Sodium chloride | | 1.0 |
| EDTA-2Na | | 0.03 |
| Purified water | | Balance |
| Total | | 100.0 |

Texture

The composition immediately after being prepared by blending the particles was subjected to sensory evaluation by a panel test of 20 persons. The persons were instructed to use the composition to evaluate comprehensively both smoothness and moist feeling, on a scale with a maximum score of 5 points according to the following criteria. The average score of the 20 persons was calculated.

Good: 5, Slightly good: 4, Fair: 3, Slightly poor: 2, and Poor: 1.

Example B-2

Preparation of sunscreen

Components shown in Table 4 were mixed, and then stirred well, and the mixture was charged into a container to prepare a sunscreen. The texture of the resulting sunscreen was evaluated by the method described above. The results are shown in Table 11.

[Table 4]

(Table 4) Example B-2

| Component | Product name, etc. | wt.% |
|---|---|---|
| Diethylaminohydroxybenzoyl hexyl benzoate | Uvinul A Plus Glanular (BASF) | 2.00 |
| Bisethylhexyloxyphenol methoxyphenyl triazine | Tnosorb S (BASF) | 0.50 |
| Ethylhexyl methoxycinnamate, BHT | Uvinul MC80 (BASF) | 7.00 |
| Diisopropyl sebacate | IPSE (Nippon Fine Chemical Co., Ltd.) | 10.00 |
| Dimethicone | KF-96A-10CS (Shin-Etsu Chemical Co., Ltd.) | 2.00 |
| Isododecane | Marukasol R (Maruzen Petrochemical Co., Ltd.) | 26.47 |
| Trimethylsiloxysilicate | MQ-1640 Flake Resin (Dow Coming Toray Co., Ltd.) | 1.00 |
| PEG-9 polydimethylsiloxyethyl dimethicone | KF-6028 (Shin-Etsu Chemical Co., Ltd.) | 2.00 |
| Titanium oxide, etc. | DIS-OP-10A (Sakai Chemical Industry Co., Ltd.) | 4.00 |
| Zinc oxide, etc. | DIF-OP-3W (Sakai Chemical Industry Co., Ltd.) | 10.00 |
| Example A-1: CA0 spherical particles | | 5.00 |
| Purified water | | 19.30 |
| BG | 1,3-BG (UK) (Daicel Corporation) | 3.00 |
| Phenoxyethanol | Phenoxyethanol-SP (Yokkaichi Chemical Co., Ltd.) | 0.20 |
| Ethanol | | 7.00 |
| Sodium chloride | | 0.50 |
| EDTA-2Na | | 0.03 |
| Total | | 100.0 |

Example B-3

Preparation of powder foundation

Component A shown in Table 5 was roughly mixed with a Henschel mixer, and then uniformly dissolved component B was added thereto, and the resultant mixture was stirred well. Thereafter, the mixture was charged into a container to prepare a powder foundation. The texture of the resulting powder foundation was evaluated by the method described above. The results are shown in Table 11.

[Table 5]

(Table 5) Example B-3

| Component | wt.% |
|---|---|
| (Component A) | |
| Example A-1: CA0 spherical particles | 7.50 |
| SI01-2 Talc JA-46R | 29.67 |
| Mica Y-2300 | 20.00 |
| SI01-2 Sericite FSE | 33.00 |
| SI01-2 Titanium oxide CR-50 | 6.50 |
| SI-2 Yellow iron oxide LLXLO | 2.30 |
| SI-2 Red iron oxide RED R-516L | 0.59 |

(continued)

| | |
|---|---|
| SI-2 Black iron oxide BL-100 | 0.44 |
| Component (A) total | 100.00 |
| (Component B) | |
| Dimethicone (20) | 20.00 |
| Dimethicone (350) | 20.00 |
| Glyceryl isostearate | 7.20 |
| Triethylhexanoin | 17.00 |
| Octyldodecyl oleate | 31.55 |
| Sorbitan stearate | 1.00 |
| Polyglyceryl-2 oleate | 3.10 |
| Propylparaben | 0.10 |
| Tocopherol | 0.05 |
| Component (B) total | 100.0 |
| (Final blending) | |
| Component A | 90.00 |
| Component B | 100.00 |

Examples B-4 to B-6 and Comparative Examples B-1 to B-2

A liquid foundation was prepared in the same manner as in Example B-1 except that the spherical particles of Example A-1 in Table 3 were changed to the particles of Examples A-4 to A-6 and Comparative Examples A-1 to A-2. The texture of each of the products was evaluated by the method described above. The results are shown in Table 11.

Example B-7

Preparation of makeup base

Component C shown in Table 6 was dispersed in component A, and the mixture was stirred well. Thereafter, component B was added thereto, and the resultant mixture was stirred and charged into a container to prepare a makeup base. The texture of the resulting makeup base was evaluated by the method described above. The results are shown in Table 11.

[Table 6]

(Table 6) Example B-7

| Component | wt.% |
|---|---|
| (Component A) | |
| (Dimethicone/(PEG-10/15)) crosspolymer, dimethicone | 3.50 |
| PEG-9 polydimethylsiloxyethyl dimethicone | 2.00 |
| Dimethicone | 5.00 |
| Isononyl isononanoate | 4.50 |
| Octyl methoxycinnamate | 10.00 |
| Quaternium-18 hectorite | 1.20 |

(continued)

| Component | wt.% |
|---|---|
| (Dimethicone/vinyl dimethicone) crosspolymer, dimethicone | 5.00 |
| Cyclomethicone | 25.00 |
| (Component B) | |
| Purified water | Balance |
| 1,3-Butylene glycol | 5.00 |
| Sodium citrate | 0.20 |
| Preservative | 0.30 |
| (Component C) | |
| Example A-1: CA0 spherical particles | 10.00 |
| Total | 100.0 |

Example B-8

Preparation of lipstick

Component B shown in Table 7 was heated to 60°C and mixed well. Component C was added thereto and dispersed well, and then component A was further added thereto and dissolved using a microwave oven, followed by mixing well. Thereafter, the mixture was dissolved again by heating using a microwave oven, poured into a mold, and solidified under cooling. This solidified product was placed in a lipstick container to prepare a lipstick. The texture of the resulting lipstick was evaluated by the method described above. The results are shown in Table 11.

[Table 7]

(Table 7) Example B-8

| Component | wt.% |
|---|---|
| (Component A) | |
| Ceresin | 4.27 |
| Microcrystalline wax | 1.55 |
| Deresinated candelilla wax | 5.03 |
| High boiling point paraffin | 3.07 |
| (Component B) | |
| Diisostearyl malate | 1.95 |
| Dipentaerythritol fatty acid ester | 6.22 |
| Adsorption refined lanolin | 2.52 |
| Liquid lanolin acetate | 13.34 |
| Glyceryl tri-2-ethylhexanoate | 19.02 |
| Liquid paraffin | 7.28 |
| Isotridecyl isononanoate | 3.21 |
| Diglyceryl triisostearate | 4.01 |
| Methylphenyl polysiloxane | 2.41 |

(continued)

| Component | wt.% |
|---|---|
| (Component A) | |
| Para-hydroxybenzoate | 0.07 |
| Natural type vitamin E | 0.05 |
| (Component C) | |
| Barium sulfate | 1.00 |
| Titanium dioxide | 3.00 |
| Red 202 | 0.50 |
| Red 201 | 0.10 |
| Red oxide | 0.20 |
| Yellow iron oxide | 0.10 |
| Example A-1: CA0 spherical particles | 10.00 |
| Total | 100.00 |

Example B-9

Preparation of face powder

Component A shown in Table 8 was mixed well using a mixer. The resulting powder was charged into a container to prepare a face powder. The texture of the resulting face powder was evaluated by the method described above. The results are shown in Table 11.

[Table 8]

(Table 8) Example B-9

| Component | wt.% |
|---|---|
| (Component A) | |
| Example A-1: CA0 spherical particles | 7.00 |
| Talc JA-46R | 44.80 |
| Mica Y-2300 | 24.00 |
| Zinc oxide FINEX-30-OTS | 18.50 |
| Propylparaben | 1.00 |
| Yellow iron oxide LLXLO | 0.70 |
| Black iron oxide BL-100 | 0.20 |
| Red iron oxide RED R-516L | 0.80 |
| Mica, titanium oxide, iron oxide 1060 T-GA | 2.00 |
| Mica, iron oxide 1060 T-WR | 1.00 |
| Component A total | 100.00 |

Example B-10

Preparation of solid face powder

Talc and a color pigment shown in Table 9 were mixed with a blender. The spherical particles (CA0 spherical particles) of Example A-1 and all powders containing the color pigment and the talc mixed with the blender in advance were stirred using a Henschel mixer. Thereafter, an oil component (binder) was added thereto, and the mixture was heated to 70°C, further stirred, and then subjected to pulverization as necessary. The resultant product was compression-molded in a gold dish container to prepare a solid face powder. The texture of the resulting solid face powder was evaluated by the method described above. The results are shown in Table 11.

[Table 9]

(Table 9) Example B-10

| Component (Powder) | wt.% |
| --- | --- |
| Talc | 30.00 |
| Sericite | 20.00 |
| Kaolin | 15.00 |
| Titanium dioxide | 5.00 |
| Zinc myristate | 5.00 |
| Magnesium carbonate | 5.00 |
| Color pigment | Suitable amount |
| Example A-1: CA0 spherical particles | 15.00 |
| (Binder) | |
| Carnauba wax | 3.00 |
| Liquid paraffin | 2.00 |
| Others: a preservative, an antioxidant, and a fragrance are added in suitable amounts as necessary. | |
| Total | 100.00 |

Example B-11

Preparation of solid powder eyeshadow

After powders shown in Table 10 were mixed well, a binder was uniformly dissolved and added to a powder portion, and the resultant mixture was further mixed. Thereafter, solid powder eyeshadow was prepared by compression molding. The texture of the resulting solid powder eyeshadow was evaluated by the method described above. The results are shown in Table 11.

[Table 10]

(Table 10) Example B-11

| Component | wt.% |
| --- | --- |
| (Powder) | |
| Mica | 15.00 |

(continued)

| Component | wt.% |
|---|---|
| (Powder) | |
| Sericite | 5.00 |
| Pigment | 15.00 |
| Pearl pigment | 10.00 |
| Example A-1: CA0 spherical particles | 51.00 |
| (Binder) | |
| Methylpolysiloxane | 2.00 |
| (Others) | |
| Sorbitan sesquioleate | 2.00 |
| Others: an antioxidant, a fragrance, and a preservative are added in a suitable amount as necessary. | |
| Total | 100.00 |

[Table 11]

| | Example B-1 | Example B-2 | Example B-3 | Example B-4 | Example B-5 | Example B-6 |
|---|---|---|---|---|---|---|
| Composition | Liquid foundation | Sunscreen | Powder foundation | Liquid foundation | Liquid foundation | Liquid foundation |
| Particles | Example A-1 | Example A-1 | Example A-1 | Example A-4 | Example A-5 | Example A6 |
| Texture | 4.2 | 4.3 | 4.3 | 4.4 | 4.1 | 3.9 |
| | Example B-7 | Example B-8 | Example B-9 | Example B-10 | Example B-11 | |
| Composition | Makeup base | Lipstick | Face powder | Solid face powder | Solid powder eyeshadow | |
| Particles | Example A-1 | Example A-1 | Example A-1 | Example A-1 | Example A-1 | |
| Texture | 4.1 | 4.4 | 4.5 | 4.1 | 4.2 | |
| | Comparative Example B-1 | Comparative Example B-2 | | | | |
| Composition | Liquid foundation | Liquid foundation | | | | |
| Particles | Comparative Example A-1 | Comparative Example A-2 | | | | |
| Texture | 2.8 | 4.1 | | | | |

As shown in Table 11, the cosmetic compositions of Examples B-1 to B-11 containing the spherical particles according to an embodiment of the present disclosure had good texture. As estimated from the results shown in Tables 1 and 2, even when the degradation reaction of the spherical particles proceeds, there is no rapid change in the texture.

Disclosure Item

Each of the following items discloses a preferred embodiment.

[Item 1]

Biodegradable spherical particles containing a biodegradable polymer as a main component, the biodegradable spherical particles having a particle size variation coefficient CV of 40% or less, and a biodegradation degree on the fifth day in a biodegradability test in accordance with OECD TG301F of 40% or less as calculated by the following formula:

$$\text{biodegradation degree (\%)} = (BOD - B)/TOD \times 100$$

(wherein BOD is a biochemical oxygen demand (mg) by a test substance, B is a biochemical oxygen demand (mg) of a blank, and TOD is a theoretical oxygen demand (mg) by the test substance).

[Item 2]

The biodegradable spherical particles according to item 1, wherein a ratio BD5/BD28 of a biodegradation degree BD5 on the fifth day to a biodegradation degree BD28 on the twenty-eighth day as measured in the biodegradability test in accordance with OECD TG301F is 0.60 or less.

[Item 3]

The biodegradable spherical particles according to item 1 or 2, wherein the biodegradation degree BD5 on the fifth day and the biodegradation degree BD28 on the twenty-eighth day as measured in the biodegradability test in accordance with OECD TG301F satisfy the following formula:

$$(BD28 - BD5)/BD5 \geq 0.50.$$

[Item 4]

The biodegradable spherical particles according to any one of items 1 to 3, wherein when a field of view of 0.5 mm $\times$ 0.5 mm is observed with a scanning electron microscope at a magnification of 5000 times, there is substantially no particle having a micron-sized recess on a surface of the particle.

[Item 5]

The biodegradable spherical particles according to any one of items 1 to 4, wherein when a field of view of 0.5 mm $\times$ 0.5 mm is observed with a scanning electron microscope at a magnification of 5000 times, there is substantially no spherical particle having a micron-sized protrusion protruding from a virtual circle drawn along an arc of the spherical particle.

[Item 6]

The biodegradable spherical particles according to any one of items 1 to 5, wherein the biodegradable polymer is selected from the group consisting of a polysaccharide, a polysaccharide ester, and an aliphatic polyester.

[Item 7]

The biodegradable spherical particles according to item 6, wherein the polysaccharide is one or two types selected from cellulose and starch.

[Item 8]

The biodegradable spherical particles according to item 6 or 7, wherein the polysaccharide ester has a total degree of substitution of more than 0 and 3.0 or less.

[Item 9]

The biodegradable spherical particles according to any one of items 6 to 8, wherein the polysaccharide ester is a cellulose acylate having an acyl group having 2 or more and 10 or less carbons, and the cellulose acylate has a total degree of substitution of more than 0 and 1.0 or less.

[Item 10]

The biodegradable spherical particles according to any one of items 6 to 9, wherein the aliphatic polyester is a polyhydroxyalkanoic acid, or a polymer of an aliphatic dicarboxylic acid and an aliphatic diol.

[Item 11]

The biodegradable spherical particles according to any one of items 6 to 10, wherein the aliphatic polyester is one or two or more types selected from the group consisting of polycaprolactone, polyhydroxybutyric acid, and polylactic acid.

[Item 12]

A cosmetic composition containing the biodegradable spherical particles described in any one of items 1 to 11.

[Item 13]

A method for producing the biodegradable spherical particles described in item 1, the method including:

preparing a mixture by mixing a biodegradable polymer, a plasticizer, and a water-soluble polymer;
melt-kneading the mixture at 200°C or higher and 280°C or lower to yield a kneaded product; and
removing the water-soluble polymer from the kneaded product.

[Item 14]

The method for producing the biodegradable spherical particles according to item 13, wherein when the biodegradable polymer is a polysaccharide ester, the method further includes mixing the kneaded product with a solvent containing one or two or more types of metal compounds selected from an alkali metal compound and an alkaline earth metal compound to hydrolyze the polysaccharide ester in removing the water-soluble polymer from the kneaded product.

[Item 15]

The method for producing the biodegradable spherical particles according to item 14, wherein the solvent is an aqueous solution of the metal compound.

[Item 16]

The method for producing the biodegradable spherical particles according to item 14 or 15, wherein the metal compound is a hydroxide of an alkali metal or an alkaline earth metal.

**Claims**

1. Biodegradable spherical particles comprising a biodegradable polymer as a main component, the biodegradable spherical particles having a particle size variation coefficient CV of 40% or less, and a biodegradation degree on the fifth day in a biodegradability test in accordance with OECD TG301F of 40% or less as calculated by the following formula:

$$\text{biodegradation degree (\%)} = (\text{BOD - B})/\text{TOD} \times 100$$

where BOD is a biochemical oxygen demand (mg) by a test substance, B is a biochemical oxygen demand (mg) of a blank, and TOD is a theoretical oxygen demand (mg) by the test substance.

2. The biodegradable spherical particles according to claim 1, wherein a ratio BD5/BD28 of a biodegradation degree BD5 on the fifth day to a biodegradation degree BD28 on the twenty-eighth day as measured in the biodegradability test in accordance with OECD TG301F is 0.60 or less.

3. The biodegradable spherical particles according to claim 1, wherein a biodegradation degree BD5 on the fifth day and a biodegradation degree BD28 on the twenty-eighth day as measured in the biodegradability test in accordance with OECD TG301F satisfy the following formula:

$$(BD28 - BD5)/BD5 \geq 0.50.$$

4. The biodegradable spherical particles according to claim 1, wherein when a field of view of 0.5 mm $\times$ 0.5 mm is observed with a scanning electron microscope at a magnification of 5000 times, there is substantially no particle having a micron-sized recess on a surface of the particle.

5. The biodegradable spherical particles according to claim 1, wherein when a field of view of 0.5 mm $\times$ 0.5 mm is observed with a scanning electron microscope at a magnification of 5000 times, there is substantially no spherical particle having a micron-sized protrusion protruding from a virtual circle drawn along an arc of the spherical particle.

6. The biodegradable spherical particles according to claim 1, wherein the biodegradable polymer is selected from the group consisting of a polysaccharide, a polysaccharide ester, and an aliphatic polyester.

7. The biodegradable spherical particles according to claim 6, wherein the polysaccharide is one or two types selected from cellulose and starch.

8. The biodegradable spherical particles according to claim 6, wherein the polysaccharide ester has a total degree of substitution of more than 0 and 3.0 or less.

9. The biodegradable spherical particles according to claim 6, wherein the polysaccharide ester is a cellulose acylate having an acyl group having 2 or more and 10 or less carbons, and the cellulose acylate has a total degree of substitution of more than 0 and 1.0 or less.

10. The biodegradable spherical particles according to claim 6, wherein the aliphatic polyester is a polyhydroxyalkanoic acid, or a polymer of an aliphatic dicarboxylic acid and an aliphatic diol.

11. The biodegradable spherical particles according to claim 6, wherein the aliphatic polyester is one or two or more types selected from the group consisting of polycaprolactone, polyhydroxybutyric acid, and polylactic acid.

12. A cosmetic composition comprising the biodegradable spherical particles described in claim 1.

13. A method for producing the biodegradable spherical particles described in claim 1, the method comprising:

preparing a mixture by mixing a biodegradable polymer, a plasticizer, and a water-soluble polymer;
melt-kneading the mixture at 200°C or higher and 280°C or lower to yield a kneaded product; and
removing the water-soluble polymer from the kneaded product.

14. The method for producing the biodegradable spherical particles according to claim 13, wherein when the biodegradable polymer is a polysaccharide ester, the method further comprises mixing the kneaded product with a solvent containing one or two or more types of metal compounds selected from an alkali metal compound and an alkaline earth metal compound to hydrolyze the polysaccharide ester in the removing the water-soluble polymer from the kneaded product.

15. The method for producing the biodegradable spherical particles according to claim 14, wherein the solvent is an aqueous solution of the metal compound.

16. The method for producing the biodegradable spherical particles according to claim 14, wherein the metal compound is a hydroxide of an alkali metal or an alkaline earth metal.

# FIG. 1

# FIG. 2

FIG. 3

# FIG. 4

FIG. 5

FIG. 6

FIG. 7

FIG. 8

## INTERNATIONAL SEARCH REPORT

| International application No. |
|---|
| **PCT/JP2023/019437** |

| **A. CLASSIFICATION OF SUBJECT MATTER** |
|---|
| *C08J 3/12*(2006.01)i; *A61K 8/02*(2006.01)i; *A61K 8/73*(2006.01)i; *A61K 8/85*(2006.01)i; *A61Q 1/00*(2006.01)i; *A61Q 1/06*(2006.01)i; *A61Q 1/10*(2006.01)i; *A61Q 17/04*(2006.01)i<br>FI: C08J3/12 Z CEP; A61K8/02; A61K8/73; A61K8/85; A61Q1/00; A61Q1/06; A61Q1/10; A61Q17/04; C08J3/12 CFD |
| According to International Patent Classification (IPC) or to both national classification and IPC |

| **B. FIELDS SEARCHED** |
|---|
| Minimum documentation searched (classification system followed by classification symbols) |
| C08J3/12; A61K8/02; A61K8/73; A61K8/85; A61Q1/00; A61Q1/06; A61Q1/10; A61Q17/04 |
| Documentation searched other than minimum documentation to the extent that such documents are included in the fields searched |
| Published examined utility model applications of Japan 1922-1996<br>Published unexamined utility model applications of Japan 1971-2023<br>Registered utility model specifications of Japan 1996-2023<br>Published registered utility model applications of Japan 1994-2023 |
| Electronic data base consulted during the international search (name of data base and, where practicable, search terms used) |
| |

| **C. DOCUMENTS CONSIDERED TO BE RELEVANT** | | |
|---|---|---|
| Category* | Citation of document, with indication, where appropriate, of the relevant passages | Relevant to claim No. |
| X | JP 2020-152851 A (DAICEL CORP.) 24 September 2020 (2020-09-24)<br>paragraphs [0059], [0060], [0089]-[0091], [0118], examples A-1, B-1, tables 1, 3 | 1-8, 12-13 |
| A | | 9-11, 14-16 |
| X | WO 2019/156116 A1 (DAICEL CORP.) 15 August 2019 (2019-08-15)<br>paragraphs [0050], [0051], [0078]-[0080], [0099], [0100], [0105]-[0110], [0118]-[0126], [0133]-[0144], examples A-1, A-7, A-9 to A-11, B-1 to B-8, B-15 to B-26, tables 1, 10 | 1-7, 12-13 |
| A | | 8-11, 14-16 |
| A | JP 2005-162841 A (DAICEL CHEM. IND., LTD.) 23 June 2005 (2005-06-23)<br>entire text | 1-16 |
| A | WO 2021/235352 A1 (DAICEL CORP.) 25 November 2021 (2021-11-25)<br>entire text | 1-16 |
| A | JP 2022-22947 A (FUJIFILM BUSINESS INNOVATION CORP.) 07 February 2022 (2022-02-07)<br>entire text | 1-16 |

✓ Further documents are listed in the continuation of Box C.   ✓ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **21 July 2023** | **01 August 2023** |
| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br><br>Telephone No. |

Form PCT/ISA/210 (second sheet) (January 2015)

## INTERNATIONAL SEARCH REPORT

| International application No. |
|---|
| **PCT/JP2023/019437** |

**C.** **DOCUMENTS CONSIDERED TO BE RELEVANT**

| Category* | Citation of document, with indication, where appropriate, of the relevant passages | Relevant to claim No. |
|---|---|---|
| A | JP 2022-7648 A (FUJIFILM BUSINESS INNOVATION CORP.) 13 January 2022 (2022-01-13)<br>entire text | 1-16 |
| A | JP 2018-172578 A (JGC CATALYSTS & CHEMICALS LTD.) 08 November 2018 (2018-11-08)<br>entire text | 1-16 |
| A | JP 2015-187255 A (FUJIFILM CORP.) 29 October 2015 (2015-10-29)<br>entire text | 1-16 |

Form PCT/ISA/210 (second sheet) (January 2015)

**INTERNATIONAL SEARCH REPORT**
Information on patent family members

| | International application No. |
|---|---|
| | **PCT/JP2023/019437** |

| Patent document cited in search report | | | Publication date (day/month/year) | Patent family member(s) | Publication date (day/month/year) |
|---|---|---|---|---|---|
| JP | 2020-152851 | A | 24 September 2020 | US 2020/0299488 A1<br>paragraphs [0062], [0063], examples A-1, B-1, tables 1, 3<br>EP 3711747 A1<br>KR 10-2020-0112641 A<br>CN 111718426 A<br>TW 202100565 A | |
| WO | 2019/156116 | A1 | 15 August 2019 | US 2020/0179261 A1<br>paragraphs [0054], [0055], [0082]-[0084], [0103], [0104], [0109]-[0113], [0121]-[0129], [0136]-[0147], examples A-1, A-7, A-9 to A-11, B-1 to B-8, B-15 to B-26, tables 1, 10<br>EP 3613794 A1<br>CN 110650994 A<br>KR 10-2019-0135537 A | |
| JP | 2005-162841 | A | 23 June 2005 | (Family: none) | |
| WO | 2021/235352 | A1 | 25 November 2021 | EP 4154944 A1<br>entire text<br>CN 115666497 A<br>TW 202207987 A | |
| JP | 2022-22947 | A | 07 February 2022 | US 2021/0403660 A1<br>entire text<br>EP 3932974 A1<br>CN 113861514 A | |
| JP | 2022-7648 | A | 13 January 2022 | (Family: none) | |
| JP | 2018-172578 | A | 08 November 2018 | US 2018/0280256 A1<br>entire text<br>CN 108685765 A<br>KR 10-2018-0111669 A | |
| JP | 2015-187255 | A | 29 October 2015 | US 2016/0355662 A1<br>entire text<br>WO 2015/137170 A1<br>CN 106062055 A | |

Form PCT/ISA/210 (patent family annex) (January 2015)

**REFERENCES CITED IN THE DESCRIPTION**

**Patent documents cited in the description**

- JP 6872068 B
- JP 2013221000 A
- JP 2004051942 A
- JP H066307 B
- WO 9204408 A

**Non-patent literature cited in the description**

- **TEZUKA.** *Carbonydr. Res.,* 1995, vol. 273 (83